# EUROPEAN PATENT SPECIFICATION

(11) **EP 3 902 788 B1**
(45) Date of publication and mention of the grant of the patent: **21.02.2024**
(21) Application number: 19845839.0
(22) Date of filing: 24.12.2019
(51) Int. Cl.: C07D 221/14, C07D 401/12, C07D 417/12, C07D 413/12, A61K 31/437, A61K 45/06, A61P 33/00, A61P 33/02, A61P 31/12, A61P 31/04, A61P 35/00

(54) **NAPHTHALIMIDE DERIVATIVES AS ANTI-PARASITIC AGENTS FOR THE TREATMENT OF LEISHMANIASIS AS WELL AS VIRAL, BACTERIAL AND NEOPLASTIC DISEASES**
NAPHTHALIMID-DERIVATE ALS ANTI-PARASITÄRE WIRKSTOFFE ZUR BEHANDLUNG VON LEISHMANIASIS SOWIE VON VIRALEN, BAKTERIELLEN UND NEOPLASTISCHEN ERKRANKUNGEN
DÉRIVÉS DE NAPHTHALIMIDE EN TANT QU'AGENTS ANTI-PARASITAIRES POUR LE TRAITEMENT DE LEISHMANIOSE AINSI QUE MALADIES VIRALES, BACTÉRIENNES ET NÉOPLASTIQUES

(30) Priority: 27.12.2018 TR 201820740
(43) Date of publication of application: 03.11.2021
(73) Proprietor: Istanbul Medipol Universitesi, Istanbul (TR); Yeditepe Universitesi, 34755 Istanbul (TR)
(72) Inventor: GUZEL, Mustafa, Istanbul (TR); ÜÇISIK, Mehmet Hikmet, Beykoz/Istanbul (TR); SAHIN, Fikrettin, Atasehir/Istanbul (TR); KESKIN, Elif, Beykoz/Istanbul (TR); ISLEK, Zeynep, Atasehir/ Istanbul (TR)
(74) Representative: Simsek, Meliha Merve
(86) International application number: PCT/TR2019/051195
(87) International publication number: WO 2020/139291

(56) References cited:
- WO-A1-2018/106200

## Description

The present invention is related to bisnaphthalimidopropyl (BNIP) derived unique drug molecules, to the usage of said unique drug molecules to treat viral, bacterial and neoplastic diseases and the preparation of a pharmaceutical composition from said unique molecules that are suitable to be used in treating viral, bacterial and neoplastic diseases.

### Prior Art

Bacterial, viral and neoplastic diseases affect a majority of the world's population and they play a crucial role in the determination of parameters such as average lifespan, life quality, etc., of people. These types of people lead to significant losses in labor power and economical burdens due to treatment expenses.

In the prior art, agents are available to treat bacterial, viral, parasitic and neoplastic diseases, however, unique agents are still required which shall provide effective treatment.

As there are not a lot of drugs in the prior art, as the application methods require medical supervision, and they cause severe side effects, makes the treatment of these diseases difficult, and this situation necessitates the development of novel active agents.

As a result of the studies carried out, it has been found that naphthalimide compounds such as mitonaphid were effective on uterine cancer and leukemia, however in the clinical studies that were carried out it has been noted that when the dose was increased these agents led to the toxicity of the central nervous system.

One of the works that is directed to development of novel naphthalimide compounds for use in treatment of bacterial, viral, parasitic or neoplastic diseases is WO 2018/106200. Said document discloses bisnaphthalimide compounds with various spacers in between the naphthalimide units.

When the prior art is taken into consideration, the invention owners aim to develop a molecule that is suitable to be used in treating viral, parasitic, neoplastic diseases that eliminate the negative aspects of known molecules such as naphthalimide derived molecules.

Another aim of the invention is to develop new molecules that do not have dose restricting toxicity problems, that are highly soluble and bioavailable contrary to the known molecules.

In accordance with these aims the invention owners who have carried out studies, have developed molecules that are suitable for treating bacterial, viral, neoplastic diseases that are shown with Formula I, Formula II, Formula III, Formula IV, Formula V, Formula VI, Formula VII, Formula VIII.

### Detailed Description of the Invention

The present invention is related to molecules of Formula I. 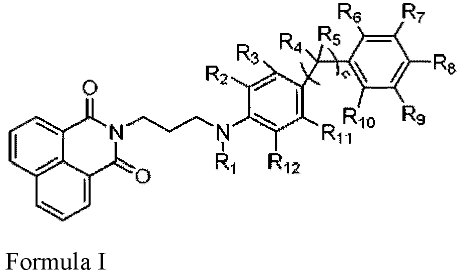 wherein
-n is a natural number between 0 to 12;
R₁, R₂, R₃, R₄, R₅, R₆, R₇, R₈, R₉, R₁₀, R₁₁, and R₁₂ are independently selected from the group of;
-H, -NH₂, -NHR_{X}, -NR_{X}R_{Y}, -NHNR_{X}, -N(R_{X})(NHR_{Y}), -N(R_{X})(NR_{Y}R_{Z}), R_{X}, R_{Y}, R_{Z}, Cl, F, Br, I, -OH, -NO, -NO₂, -COOH, -OR_{X}, -SO₂R_{X}, -SO₂NHR_{X}, -SO₂NR_{X}R_{Y}, -CN, -COOR_{X}, -CONHR_{X}, -CONR_{X}R_{Y}, -NHCOCH₂R_{X}, -NHCOCHR_{X}R_{Y}, -NHCNHR_{X}, -NHCOR_{X}, -NHCONHRx, -NHSO₂R_{X} and;
R_{X}, R_{Y}, and R_{Z} are independently selected from substituted or unsubstituted C1-C12 alkane, substituted or unsubstituted C1-C12 alkene, substituted or unsubstituted C1-C12 alkyne, substituted or unsubstituted C1-C12 primary amine, substituted or unsubstituted C1-C12 secondary amine, substituted or unsubstituted alcohol, substituted or unsubstituted alkyl cyanide, substituted or unsubstituted sulphonamide, substituted or unsubstituted C1-C12 alkyl sulphonyl, substituted or unsubstituted C3-C8 cycloalkyl, substituted or unsubstituted aryl, substituted or unsubstituted heteroaryl, optionally and independent from each other from a group formed of substituted or unsubstituted heterocyclic or heteroaromatic rings that comprise one or more heteroatoms selected from O, S and N.

The term "alkane" as used within the scope of the invention, defines straight or branched saturated hydrocarbon chains.

The term "alkene" as used within the scope of the invention, defines straight or branched hydrocarbon chains that comprise at least a carbon-carbon double bond.

The term "alkyne" as used within the scope of the invention, defines straight or branched hydrocarbon chains that comprise at least a carbon-carbon triple bond.

According to a preferred embodiment of the invention the molecules according to the invention are illustrated with Formula I; 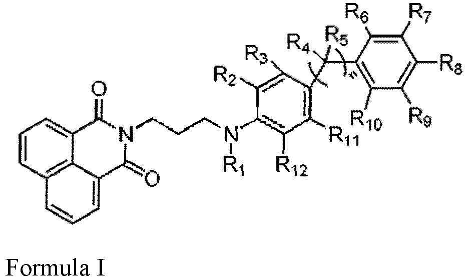
n takes the value of 0, or 1 or 2 or 3 or 4 or 5 or 6 or 7 or 8 or 9 or 10 or 11 or 12;
R₁, R₂, R₃, R₄, R₅, R₆, R₇, R₈, R₉, R₁₀, R₁₁ and R₁₂ are independently selected from the group of;
-H, -NH₂, -CH₃, -CH₂CH₃, -CH₂CH₂CH₃, -CH₂CH₂CH₂CH₃, -CH₂CH₂CH₂CH₂CH₃, -CH₂CH₂CH₂CH₂CH₂CH₃, -CH₂CH₂CH₂CH₂CH₂CH₂CH₃, - CH₂CH₂CH₂CH₂CH₂CH₂CH₂CH₃, -CH₂CH₂CH₂CH₂CH₂CH₂CH₂CH₂CH₃, CH₂CH₂CH₂CH₂CH₂CH₂CH₂CH₂CH₂CH₃, - CH₂CH₂CH₂CH₂CH₂CH₂CH₂CH₂CH₂CH₂CH₃, -CH₂CH₂CH₂CH₂CH₂CH₂CH₂CH₂CH₂CH₂CH₂CH₃, -C(CH₃)₃, Cl, F, Br, I, -OH, -OCH₃, - OCH₂CH₃, -OCH₂CH₂CH₃, -OCH₂CH₂CH₂CH₃, -OCH₂CH₂CH₂CH₂CH₃, - OCH₂CH₂CH₂CH₂CH₂CH₃, -NHCH₃, -NHCH₂CH₃, -NHCH₂CH₂CH₃, - NHCH₂CH₂CH₂CH₃, -NHCH₂CH₂CH₂CH₂CH₃, -NHCH₂CH₂CH₂CH₂CH₂CH₃, - N(CH₃)₂, -N(CH₂CH₃)(CH₃), -N(CH₂CH₃)₂, -N(CH₂CH₃)(CH₂CH₂CH₃), - N(CH₃)(CH₂CH₂CH₃), N(CH₂CH₂CH₃)₂, -NHNH₂, -NHNHCH₃, -NHNHCH₂CH₃, - NHNHCH₂CH₂CH₃, -NHN(CH₃)₂, -NHN(CH₂CH₃)₂, -NHN(CH₃)(CH₂CH₃), - N(CH₃)(NHCH₃), -N(CH₃)(N(CH₃)₂), -COOH, -SO₂CH₃, -SO₂CH₂CH₂, - SO₂CH₂CH₂CH₃, -SO₂CH₂CH₂CH₂CH₃, -SO₂CH₂CH₂CH₂CH₂CH₃, - SO₂CH₂CH₂CH₂CH₂CH₂CH₃, -CN, -CONH₂, -CONHCH₃, -CONHCH₂CH₃, - CONHCH₂CH₂CH₃, -CONHCH₂CH₂CH₂CH₃, -CONHCH₂CH₂CH₂CH₂CH₃, - CONHCH₂CH₂CH₂CH₂CH₂CH₃, -CON(CH₃)₂, -CON(CH₃)(CH₂CH₃), - CON(CH₂CH₃)₂, -CON(CH₂CH₃)(CH₂CH₂CH₃), -CON(CH₃)(CH₂CH₂CH₃), - CON(CH₂CH₂CH₃)₂, -COCH₃, -COCH₂CH₃, -COCH₂CH₂CH₃, - COCH₂CH₂CH₂CH₃, -COCH₂CH₂CH₂CH₂CH₃, -COCH₂CH₂CH₂CH₂CH₂CH₃, - COOCH₃, -COOCH₂CH₃, -COOCH₂CH₂CH₃, -COOCH₂CH₂CH₂CH₃, - COOCH₂CH₂CH₂CH₂CH₃, -COOCH₂CH₂CH₂CH₂CH₂CH₃, -COCH(CH₃)₂, - COC(CH₃)₃, -COCH(CH₂CH₃)₂, -COCH(CH₃)(CH₂CH₃), -COCH₂CH₂CH₃, - COCH₂CH₂CH₂CH₃, -COCH₂CH₂CH₂CH₂CH₃, -COCH₂CH₂CH₂CH₂CH₂CH₃, - SO₂NH₂, SO₂NHCH₃, SO₂NHCH₂CH₃, -SO₂NHCH₂CH₂CH₃, SO₂N(CH₃)₂, - SO₂N(CH₃)(CH₂CH₃), -SO₂N(CH₂CH₃)₂, -SO₂N(CH₃)(CH₂CH₂CH₃), - SO₂N(CH₂CH₂CH₃)₂, -NHCONHCH₃, -NHCON(CH₃)₂, -NHCONHCH₂CH₃, - NHCON(CH₃)(CH₂CH₃), -NHCON(CH₂CH₃)₂, -NHCOCH(CH₃)(NH₂), - NHCOCH(CH₃)₂, -NHCOCH(CH₃)(NHCH₃), -NHCOCH(CH₃)(N(CH₃)₂), - NHCOCH(CH₂CH₃)₂, -NHCOCH(CH₂OH)(NH₂), -NHCOCH(CH₂CH₂OH)(NH₂), - NHCOCH(CH₂CH₂OH)(NHCH₃), -NHCOCH(CH₂OH)(NHCH₃), NHCOCH(CH₂OH)(N(CH₃)₂), NHCOCH(CH₂OH)(N(CH₂CH₃)₂), - NHCOCH(CH₂SH)(NH₂), -NHCOCH(CH₂CH₂SH)(NH₂), - NHCOCH(CH₂CH₂CH₂SH)(NH₂), -NHCOCH(CH₂SH)(NHCH₃), - NHCOCH(CH₂SH)(NHCH₂CH₃), -NHCOCH(CH₂SH)(N(CH₃)₂), 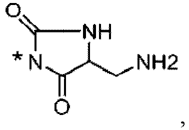 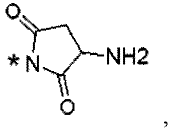 -NH(CNH)NH₂, -NH(CN(CH₃))NH₂, -NH(CN(CH₂CH₃))NH₂, - NH(CN(CH₃))NH(CH₃), - NH(CN(CH₃))N(CH₃)₂, -NH(CNH)NHCH₃, NH(CNH)N(CH₃)₂, -NH(CO)NHCH₂CH₂NH₂, -NH(CO)NHCH₂NH₂, - NH(CO)NHCH₂CH₂NHCH₃, -NH(CO)N(CH₃)(CH₂CH₂NH₂), - NH(CO)CH₂CH₂NH₂, -NH(CO)CH₂NH₂, -NH(CO)CH₂CH₂CH₂NH₂, - NH(CO)CH₂CH₂CH₂CH₂NH₂, -NH(CO)CH₂CH₂NHCH₃, -NH(CO)CH₂CH₂N(CH₃)₂, -NH(CO)CH₂CH₂NH(CH₂CH₃), -NH(CO)CH₂CH₂N(CH₃)(CH₂CH₃), - NHSO₂CH₂CH₂NH₂, -NHSO₂CH₂CH₂CH₂NH₂, -NHSO₂CH₂CH₂NHCH₃, - NHSO₂CH₂CH₂N(CH₃)₂, -NHSO₂CH₂CH₂NH₂(CH3)(CH₂CH₃), - NHSO₂CH₂CH₂N(CH₂CH₃)(CH₂CH₃), -NHCH₂CH₂NH₂, -NHCH₂NH₂, - NHCH₂CH₂CH₂NH₂, -NHCH₂CH₂CH₂CH₂NH₂, -NHCH₂CH₂NHCH₃, - NHCH₂CH₂N(CH₃)₂, -N(CH₂CH₂NH₂)₂, -N(CH₂CH₂CH₂NH₂)₂, -N(CH₂NH₂)₂, - N(CH₂NHCH₃)₂, -N(CH₂CH₂NHCH₃)₂, -N(CH₂CH₂CH₂NHCH₃)₂, - NHCONHCH₂CH₂SO₂NH₂, -NHCONHCH₂CH₂CH₂SO₂NH₂, - NHCON(CH3)(CH₂CH₂CH₂SO₂NH₂), -NHCON(CH₂CH₃)(CH₂CH₂CH₂SO₂NH₂), - NHCON(CH3)(CH₂CH₂SO₂NH₂), -NHCONHCH₂CH₂SO₂NHCH₃, - NHCONHCH₂CH₂SO₂NHCH₂CH₃, - NHCONHCH₂CH₂SO₂N(CH₃)₂, - NHCH₂CH₂SO₂NH₂, -NHCH₂CH₂CH₂SO₂NH₂, - NHCH₂CH₂CH₂CH₂SO₂NH₂, - NHCH₂CH₂SO₂NHCH₃, -NHCH₂CH₂SO₂N(CH₃)₂.

Another aspect of the invention are molecules shown with Formula II

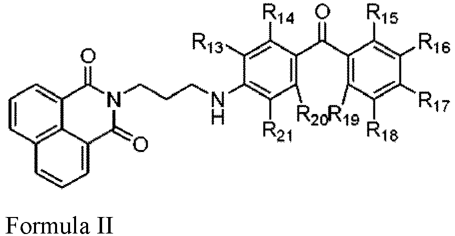

Wherein R₁₃, R₁₄, R₁₅, R₁₆, R₁₇, R₁₈, R₁₉, R₂₀, R₂₁ are independently selected from; -H, -Cl, -F, -Br, -I, R_{X}, -NH₂, -NHR_{X}, -NR_{X}R_{Y}, -OH, -NO, -NO₂, -COOH, -OR_{X}, -COOR_{X}, - CN and
R_{X} and R_{Y} are independently selected from substituted or unsubstituted C1-C12 alkane, substituted or unsubstituted C1-C12 alkene, substituted or unsubstituted C1-C12 alkyne, substituted or unsubstituted C1-C12 primary amine, substituted or unsubstituted C1-C12 secondary amine, substituted or unsubstituted alcohol, substituted or unsubstituted alkyl cyanide, substituted or unsubstituted sulphonamide, substituted or unsubstituted C1-C12 alkyl sulphonyl, substituted or unsubstituted C3-C8 cycloalkyl, substituted or unsubstituted aryl, substituted or unsubstituted heteroaryl, optionally and independent from each other from a group formed of substituted or unsubstituted heterocyclic or hetero-aromatic rings that comprise one or more heteroatoms selected from O, S and N.

According to a preferred embodiment of the invention the molecules according to the invention are illustrated with Formula II;

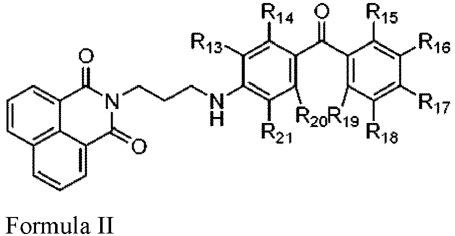

Wherein R₁₃, R₁₄, R₁₅, R₁₆, R₁₇, R₁₈, R₁₉, R₂₀, R₂₁ are independently selected from the group of;-H, -Cl, -F, -Br, -I, -NH₂, -CH₃, C(CH₃)₂. Another aspect of the invention are molecules shown with Formula III

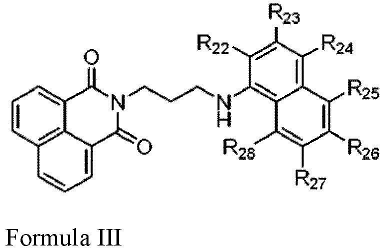

Wherein R₂₂, R₂₃, R₂₄, R₂₅, R₂₆, R₂₇, R₂₈, are independently selected from the group of; -H, -Cl, -F, -Br, -I, R_{X}, -NH₂, -NHR_{X}, -NR_{X}R_{Y}, -OH, -COOH, -OR_{X}, -COOR_{X}, -CN, - SO₃H, -SO₃⁻Na⁺, 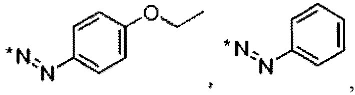 -NO and
R_{X} and R_{Y} are independently selected from substituted or unsubstituted C1-C12 alkane, substituted or unsubstituted C1-C12 alkene, substituted or unsubstituted C1-C12 alkyne, substituted or unsubstituted C1-C12 primary amine, substituted or unsubstituted C1-C12 secondary amine, substituted or unsubstituted alcohol, substituted or unsubstituted alkyl cyanide, substituted or unsubstituted sulphonamide, substituted or unsubstituted C1-C12 alkyl sulphonyl, substituted or unsubstituted C3-C8 cycloalkyl, substituted or unsubstituted aryl, substituted or unsubstituted heteroaryl, optionally and independent from each other from a group formed of substituted or unsubstituted heterocyclic or heteroaromatic rings that comprise one or more heteroatoms selected from O, S and N.

Another aspect of the invention are molecules shown with Formula IV

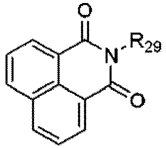

Formula IV is selected from the group formed of R₂₉

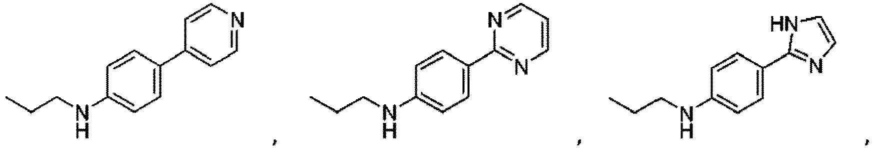

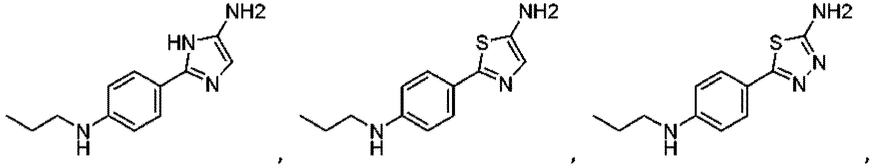

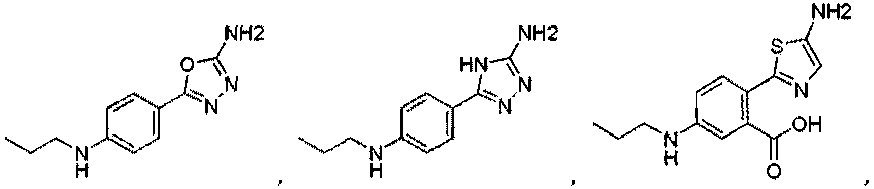

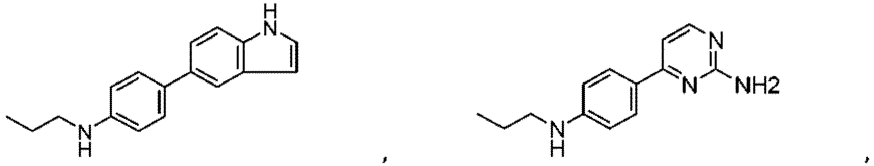

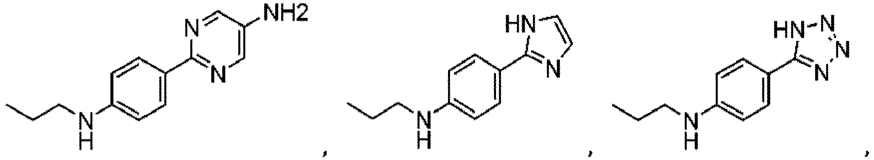

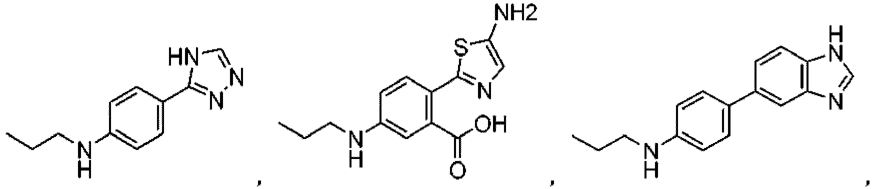

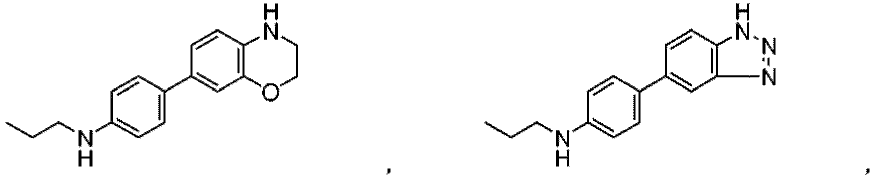

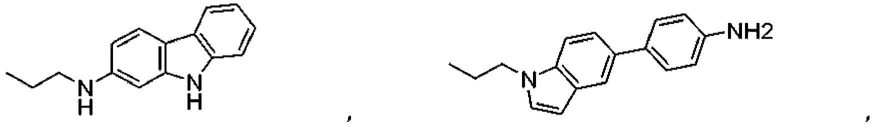

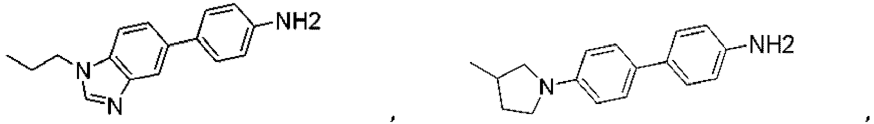

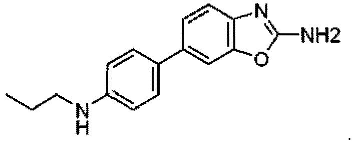

Another aspect of the invention are molecules shown with Formula V

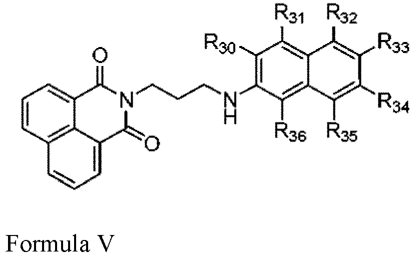

Wherein R₃₀, R₃₁, R₃₂, R₃₃, R₃₄, R₃₅, R₃₆ are independently selected from the group of;
-H, -Cl, -F, -Br, -I, R_{X}, -NH₂, -NHR_{X}, -NR_{X}R_{Y}, -OH, -NO, -COOH, -OR_{X}, -COOR_{X}, -CN and
R_{X} and R_{Y} are independently selected from substituted or unsubstituted C1-C12 alkane, substituted or unsubstituted C1-C12 alkene, substituted or unsubstituted C1-C12 alkyne, substituted or unsubstituted C1-C12 primary amine, substituted or unsubstituted C1-C12 secondary amine, substituted or unsubstituted alcohol, substituted or unsubstituted alkyl cyanide, substituted or unsubstituted sulphonamide, substituted or unsubstituted C1-C12 alkyl sulphonyl, substituted or unsubstituted C3-C8 cycloalkyl, substituted or unsubstituted aryl, substituted or unsubstituted heteroaryl, optionally and independent from each other from a group formed of substituted or unsubstituted heterocyclic or heteroaromatic rings that comprise one or more heteroatoms selected from O, S and N.

According to a preferred embodiment of the invention the molecules according to the invention are illustrated with Formula V; 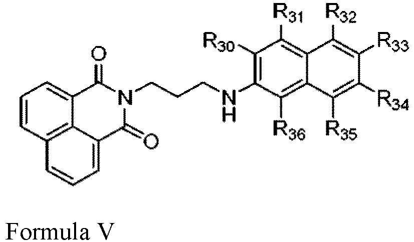 wherein R₃₀, R₃₁, R₃₂, R₃₃, R₃₄, R₃₅, R₃₆ are independently selected from the group of; -H, -NH₂, -OCH3, -NO, -Cl, F, I, Br.

Another aspect of the invention are molecules shown with Formula VI; 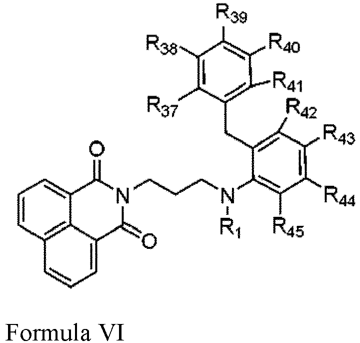
wherein R₁, R₃₇, R₃₈, R₃₉, R₄₀, R₄₁, R₄₂, R₄₃, R₄₄, R₄₅ are independently selected from the group of;
-H, -NH₂, -NHR_{X}, -NR_{X}R_{Y}, -NHNR_{X}, -N(R_{X})(NHR_{Y}), -N(R_{X})(NR_{Y}R_{Z}), R_{X}, R_{Y}, R_{Z}, Cl, F, Br, I, -OH, -NO, -NO₂, -COOH, -OR_{X}, -SO₂R_{X}, -SO₂NHR_{X}, -SO₂NR_{X}R_{Y}, -CN, -COOR_{X}, - CONHR_{X}, -CONR_{X}R_{Y}, -NHCOCH₂R_{X}, -NHCOCHR_{X}R_{Y}, -NHCNHR_{X}, -NHCOR_{X}, - NHCONHRx, -NHSO₂R_{X} and;
R_{X}, R_{Y}, and R_{Z} are independently selected from substituted or unsubstituted C1-C12 alkane, substituted or unsubstituted C1-C12 alkene, substituted or unsubstituted C1-C12 alkyne, substituted or unsubstituted C1-C12 primary amine, substituted or unsubstituted C1-C12 secondary amine, substituted or unsubstituted alcohol, substituted or unsubstituted alkyl cyanide, substituted or unsubstituted sulphonamide, substituted or unsubstituted C1-C12 alkyl sulphonyl, substituted or unsubstituted C3-C8 cycloalkyl, substituted or unsubstituted aryl, substituted or unsubstituted heteroaryl, optionally and independent from each other from a group formed of substituted or unsubstituted heterocyclic or heteroaromatic rings that comprise one or more heteroatoms selected from O, S and N.

According to a preferred embodiment of the invention the molecules according to the invention are illustrated with Formula VI; 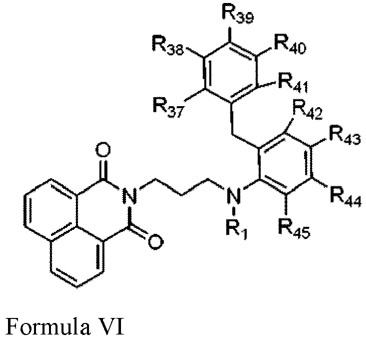
wherein R₁, R₃₇, R₃₈, R₃₉, R₄₀, R₄₁, R₄₂, R₄₃, R₄₄, R₄₅ are independently selected from the group of;
-H, -NH₂, -CH₃, -CH₂CH₃, -CH₂CH₂CH₃, -C(CH₃)₃, Cl, F, Br, I, -OH, -OCH₃, -OCH₂CH₃, - NHCH₃, -NHCH₂CH₃, -N(CH₃)₂, -NO₂, -COOH, -COOCH₃, -COONH₂, -COONHCH₃, - COON(CH₃)₂.

Another aspect of the invention are molecules shown with Formula VII; 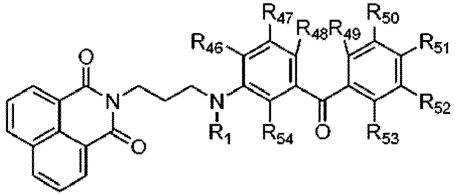 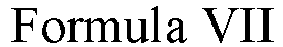
where R₁, R₄₆, R₄₇, R₄₈, R₄₉, R₅₀, R₅₁, R₅₂, R₅₃, R₅₄ is selected from the group of;
-H, -NH₂, -NHR_{X}, -NR_{X}R_{Y}, -NHNR_{X}, -N(R_{X})(NHR_{Y}), -N(R_{X})(NR_{Y}R_{Z}), R_{X}, R_{Y}, R_{Z}, Cl, F, Br, I, -OH, -NO, -NO₂, -COOH, -OR_{X}, -SO₂R_{X}, -SO₂NHR_{X}, -SO₂NR_{X}R_{Y}, -CN, -COOR_{X}, - CONHR_{X}, -CONR_{X}R_{Y}, -NHCOCH₂R_{X}, -NHCOCHR_{X}R_{Y}, -NHCNHR_{X}, -NHCOR_{X}, - NHCONHRx, -NHSO₂R_{X} and;
R_{X}, R_{Y}, and R_{Z} are independently selected from substituted or unsubstituted C1-C12 alkane, substituted or unsubstituted C1-C12 alkene, substituted or unsubstituted C1-C12 alkyne, substituted or unsubstituted C1-C12 primary amine, substituted or unsubstituted C1-C12 secondary amine, substituted or unsubstituted alcohol, substituted or unsubstituted alkyl cyanide, substituted or unsubstituted sulphonamide, substituted or unsubstituted C1-C12 alkyl sulphonyl, substituted or unsubstituted C3-C8 cycloalkyl, substituted or unsubstituted aryl, substituted or unsubstituted heteroaryl, optionally and independent from each other from a group formed of substituted or unsubstituted heterocyclic or heteroaromatic rings that comprise one or more heteroatoms selected from O, S and N.

According to a preferred embodiment of the invention the molecules according to the invention are illustrated with Formula VII; 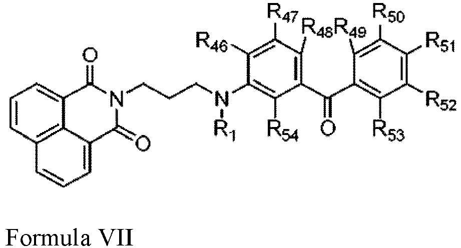
wherein R₁, R₄₆, R₄₇, R₄₈, R₄₉, R₅₀, R₅₁, R₅₂, R₅₃, R₅₄ is selected from the group of;
-H, -NH₂, -CH₃, -CH₂CH₃, -CH₂CH₂CH₃, -C(CH₃)₃, Cl, F, Br, I, -OH, -OCH₃, -OCH₂CH₃, - NHCH₃, -NHCH₂CH₃, -N(CH₃)₂, -NO₂, -COOH, -COOCH₃, -COONH₂, -COONHCH₃, - COON(CH₃)₂.

According to a preferred embodiment of the invention R₁ in the molecules of Formula VII is - H.

Another aspect of the invention are molecules shown with Formula VIII; 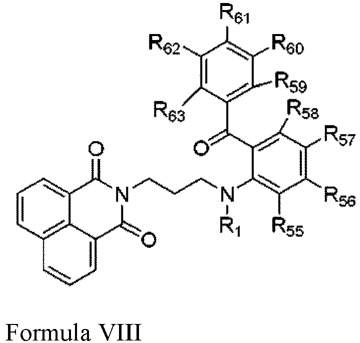
wherein R₁, R₅₅, R₅₆, R₅₇, R₅₈, R₅₉, R₆₀, R₆₁, R₆₂, R₆₃ are independently selected from the group of;
-H, -NH₂, -NHR_{X}, -NR_{X}R_{Y}, -NHNR_{X}, -N(R_{X})(NHR_{Y}), -N(R_{X})(NR_{Y}R_{Z}), R_{X}, R_{Y}, R_{Z}, Cl, F, Br, I, -OH, -NO, -NO₂, -COOH, -OR_{X}, -SO₂R_{X}, -SO₂NHR_{X}, -SO₂NR_{X}R_{Y}, -CN, -COOR_{X}, - CONHR_{X}, -CONR_{X}R_{Y}, -NHCOCH₂R_{X}, -NHCOCHR_{X}R_{Y}, -NHCNHR_{X}, -NHCOR_{X}, - NHCONHRx, -NHSO₂R_{X} and;
R_{X}, R_{Y}, and R_{Z} are independently selected from substituted or unsubstituted C1-C12 alkane, substituted or unsubstituted C1-C12 alkene, substituted or unsubstituted C1-C12 alkyne, substituted or unsubstituted C1-C12 primary amine, substituted or unsubstituted C1-C12 secondary amine, substituted or unsubstituted alcohol, substituted or unsubstituted alkyl cyanide, substituted or unsubstituted sulphonamide, substituted or unsubstituted C1-C12 alkyl sulphonyl, substituted or unsubstituted C3-C8 cycloalkyl, substituted or unsubstituted aryl, substituted or unsubstituted heteroaryl, optionally and independent from each other from a group formed of substituted or unsubstituted heterocyclic or heteroaromatic rings that comprise one or more heteroatoms selected from O, S and N.

According to a preferred embodiment of the invention the molecules according to the invention are illustrated with Formula VIII; 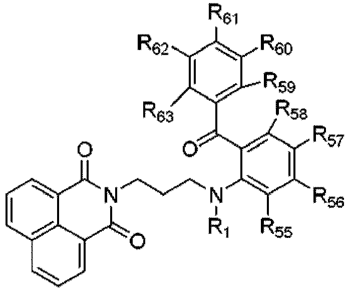 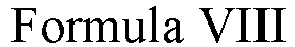
wherein R₁, R₅₅, R₅₆, R₅₇, R₅₈, R₅₉, R₆₀, R₆₁, R₆₂, R₆₃ are independently selected from the group of;
-H, -NH₂, -CH3, -CH₂CH₃, -CH₂CH₂CH₃, -C(CH3)3, Cl, F, Br, I, -OH, -OCH3, -OCH₂CH₃, - NHCH₃, -NHCH₂CH₃, -N(CH₃)₂, -NO₂, -COOH, -COOCH3, -COONH₂, -COONHCH₃, - COON(CH₃)₂.

According to a preferred embodiment of the invention R₁ in the molecules of Formula VIII is -H.

The present invention is related to bisnaphthalimidopropyl derived molecules, wherein molecules of the invention are shown with;
a) Formula I; 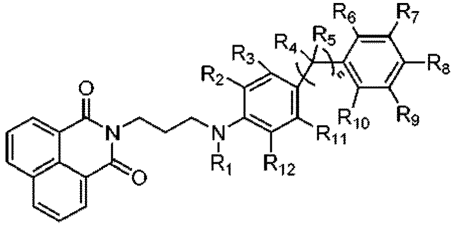
b) Formula II; 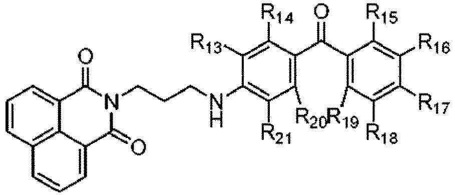
c) Formula III; 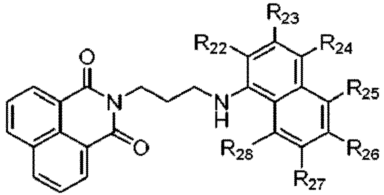
d) Formula IV; 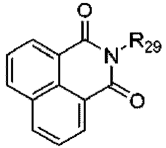
e) Formula V; 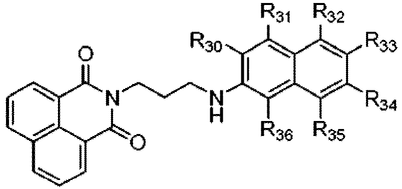
f) Formula VI; 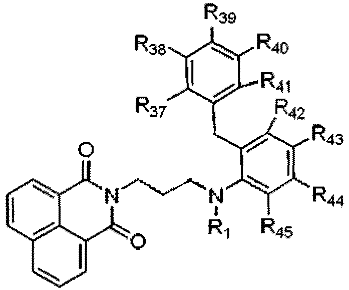
g) Formula VII; 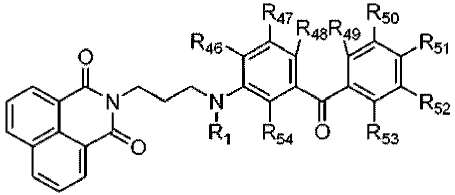
h) Formula VIII; 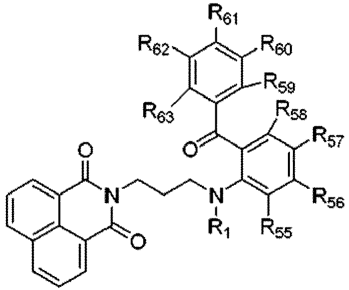 wherein
   -n is a natural number between 0 to 12,
   - R₁, R₂, R₃, R₄, R₅, R₆, R₇, R₈, R₉, R₁₀, R₁₁, R₁₂, R₃₇, R₃₈, R₃₉, R₄₀, R₄₁, R₄₂, R₄₃, R₄₄, R₄₅, R₄₆, R₄₇, R₄₈, R₄₉, R₅₀, R₅₁, R₅₂, R₅₃, R₅₄, R₅₅, R₅₆, R₅₇, R₅₈, R₅₉, R₆₀, R₆₁, R₆₂, R₆₃,
   are independently selected from the group of;
   -H, -NH₂, -NHR_{X}, -NR_{X}R_{Y}, -NHNR_{X}, -N(R_{X})(NHR_{Y}), -N(R_{X})(NR_{Y}R_{Z}), R_{X}, R_{Y}, R_{Z}, Cl, F, Br, I, -OH, -NO, -NO₂, -COOH, -OR_{X}, -SO₂R_{X}, -SO₂NHR_{X}, -SO₂NR_{X}R_{Y}, -CN, -COOR_{X}, - CONHR_{X}, -CONR_{X}R_{Y}, -NHCOCH₂R_{X}, -NHCOCHR_{X}R_{Y}, -NHCNHR_{X}, -NHCOR_{X}, - NHCONHRx, -NHSO₂R_{X};
   - R₁₃, R₁₄, R₁₅, R₁₆, R₁₇, R₁₈, R₁₉, R₂₀, R₂₁ is independently selected from;
   -H, -Cl, -F, -Br, -I, R_{X}, -NH₂, -NHR_{X}, -NR_{X}R_{Y}, -OH, -NO, -NO₂, -COOH, -OR_{X}, -COOR_{X}, - CN;
   - R₂₂, R₂₃, R₂₄, R₂₅, R₂₆, R₂₇, R₂₈ are independently selected from;
   -H, -Cl, -F, -Br, -I, R_{X}, -NH₂, -NHR_{X}, -NR_{X}R_{Y}, -OH, -COOH, -OR_{X}, -COOR_{X}, -CN, - SO₃H, -SO₃⁻Na⁺, 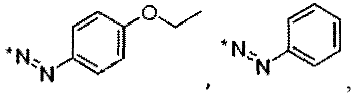 -NO;
   -R₂₉ is selected from the group of; 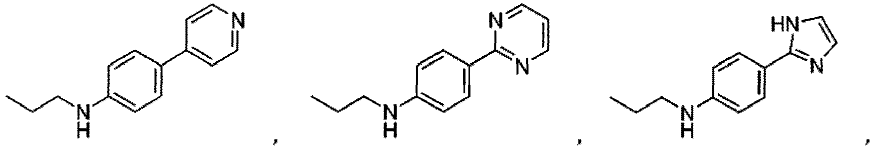 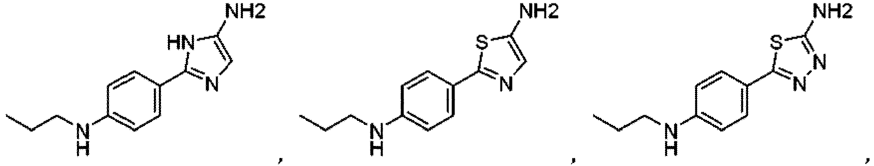 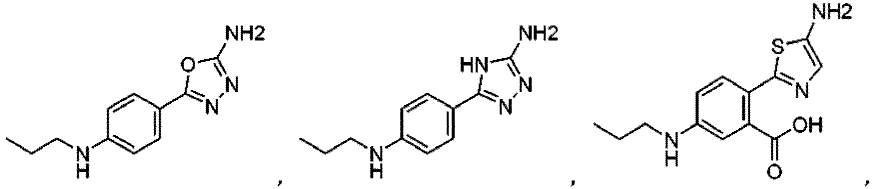 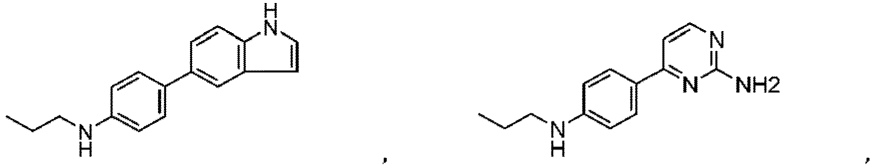 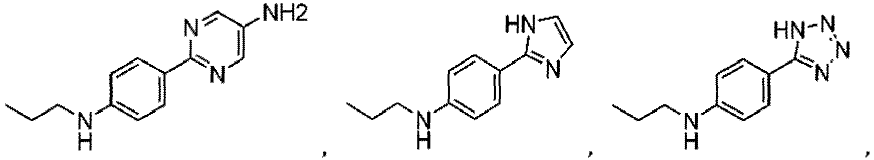 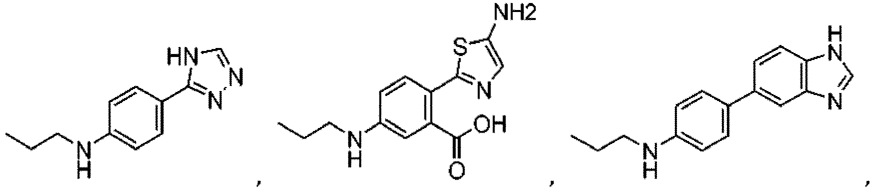 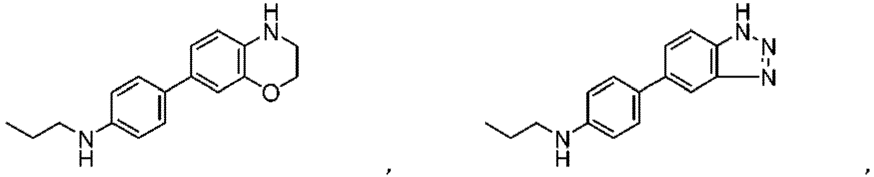 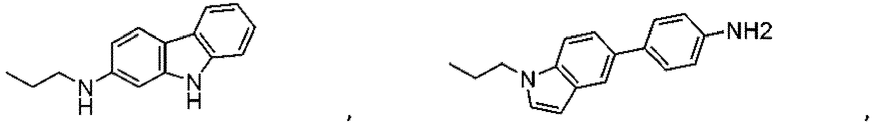 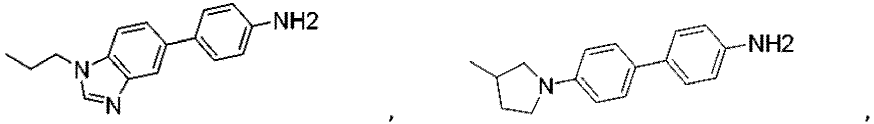 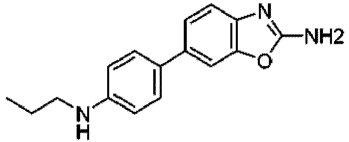
   - R₃₀, R₃₁, R₃₂, R₃₃, R₃₄, R₃₅, R₃₆ are independently selected from the group of;
   -H, -Cl, -F, -Br, -I, R_{X}, -NH₂, -NHR_{X}, -NR_{X}R_{Y}, -OH, -NO, -COOH, -OR_{X}, -COOR_{X},-CN;
   R_{X}, R_{Y}, and R_{Z} are independently selected from substituted or unsubstituted C1-C12 alkane, substituted or unsubstituted C1-C12 alkene, substituted or unsubstituted C1-C12 alkyne, substituted or unsubstituted C1-C12 primary amine, substituted or unsubstituted C1-C12 secondary amine, substituted or unsubstituted alcohol, substituted or unsubstituted alkyl cyanide, substituted or unsubstituted sulphonamide, substituted or unsubstituted C1-C12 alkyl sulphonyl, substituted or unsubstituted C3-C8 cycloalkyl, substituted or unsubstituted aryl, substituted or unsubstituted heteroaryl, optionally and independent from each other from a group formed of substituted or unsubstituted heterocyclic or heteroaromatic rings that comprise one or more heteroatoms selected from O, S and N.

According to a preferred embodiment of the invention the molecule of Formula I is Formula 1;

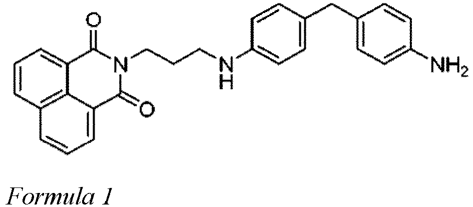

According to a preferred embodiment of the invention the molecule of Formula I is Formula 2;

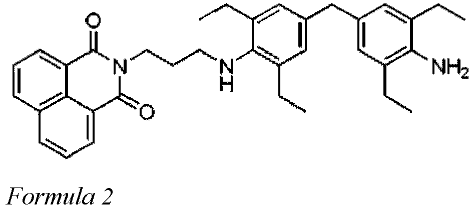

According to a preferred embodiment of the invention the molecule of Formula I is Formula 3;

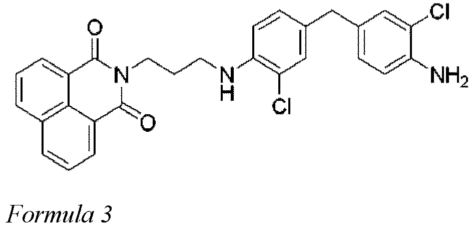

According to a preferred embodiment of the invention the molecule of Formula I is Formula 4;

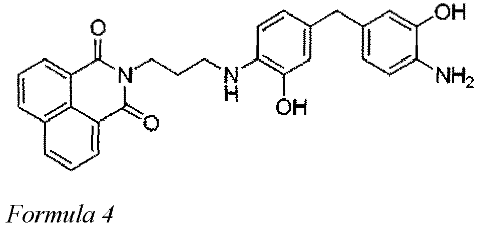

According to a preferred embodiment of the invention the molecule of Formula I is Formula 5;

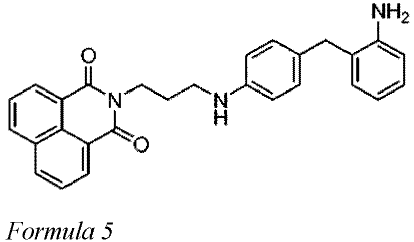

According to a preferred embodiment of the invention the molecule of Formula I is Formula 6;

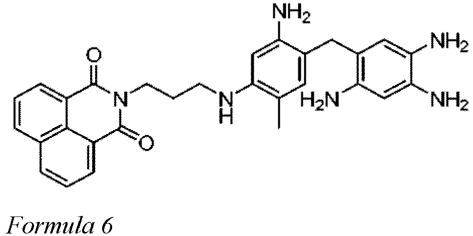

According to a preferred embodiment of the invention the molecule of Formula I is Formula 7;

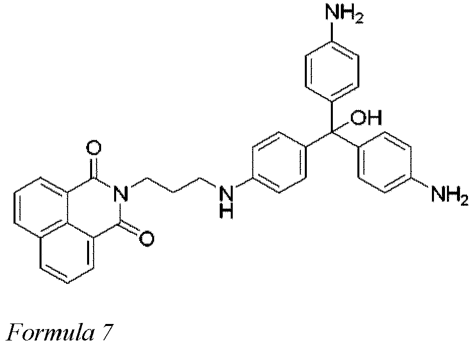

According to a preferred embodiment of the invention the molecule of Formula I is Formula 8;

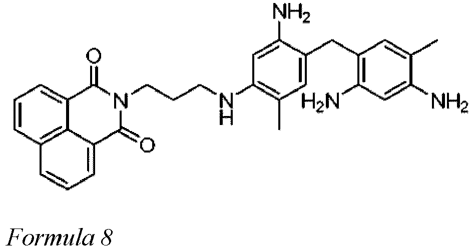

According to a preferred embodiment of the invention the molecule of Formula I is Formula 9;

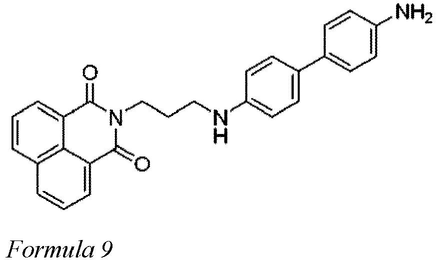

According to a preferred embodiment of the invention the molecule of Formula I is Formula 10;

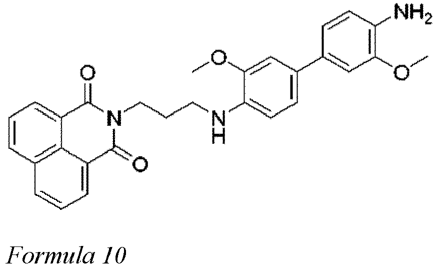

According to a preferred embodiment of the invention the molecule of Formula I is Formula 11;

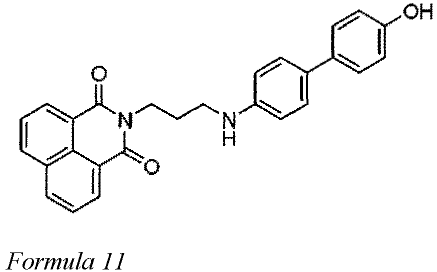

According to a preferred embodiment of the invention the molecule of Formula I is Formula 12;

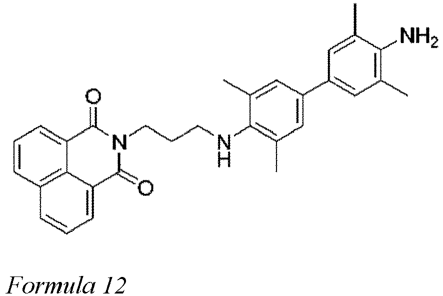

According to a preferred embodiment of the invention the molecule of Formula I is Formula 13;

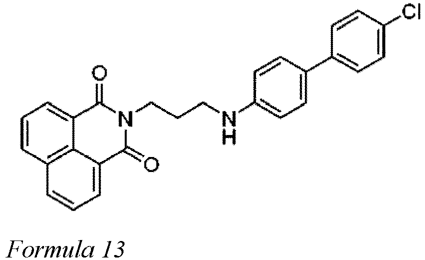

According to a preferred embodiment of the invention the molecule of Formula I is Formula 14;

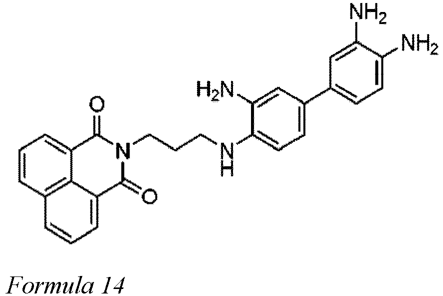

According to a preferred embodiment of the invention the molecule of Formula I is Formula 15;

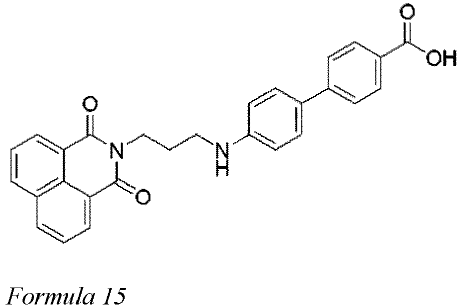

According to a preferred embodiment of the invention the molecule of Formula I is Formula 16;

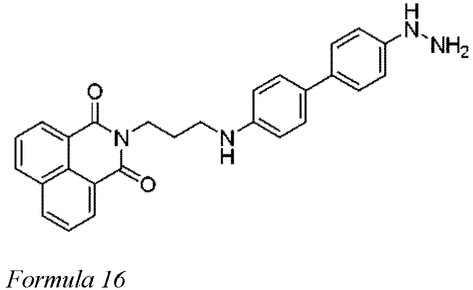

According to a preferred embodiment of the invention the molecule of Formula I is Formula 17;

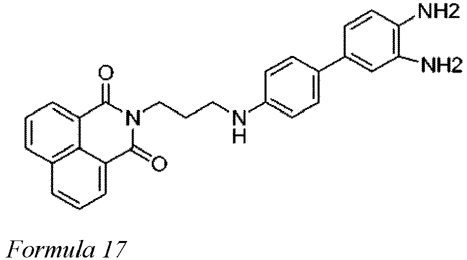

According to a preferred embodiment of the invention the molecule of Formula I is Formula 18;

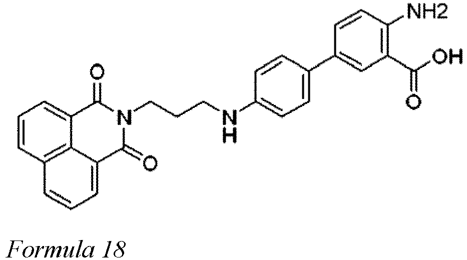

According to a preferred embodiment of the invention the molecule of Formula I is Formula 19;

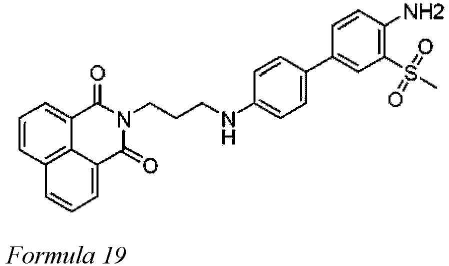

According to a preferred embodiment of the invention the molecule of Formula I is Formula 20;

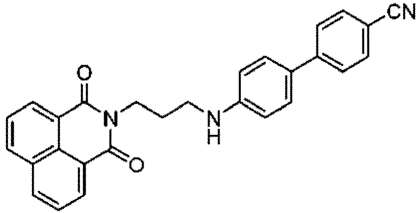

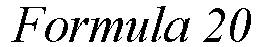

According to a preferred embodiment of the invention the molecule of Formula I is Formula 21;

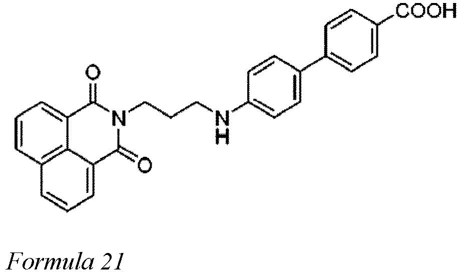

According to a preferred embodiment of the invention the molecule of Formula I is Formula 22;

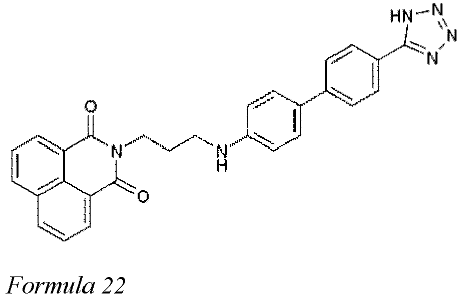

According to a preferred embodiment of the invention the molecule of Formula I is Formula 23;

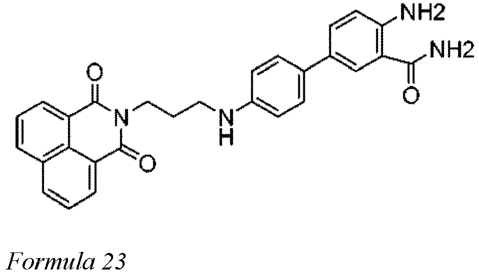

According to a preferred embodiment of the invention the molecule of Formula I is Formula 24;

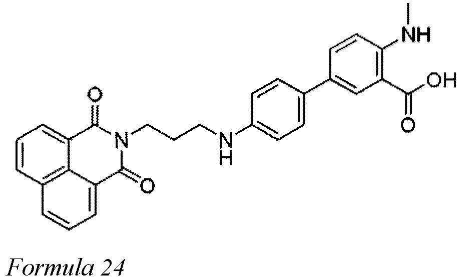

According to a preferred embodiment of the invention the molecule of Formula I is Formula 25;

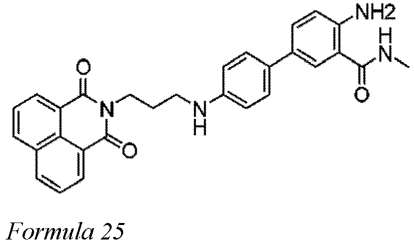

According to a preferred embodiment of the invention the molecule of Formula I is Formula 26;

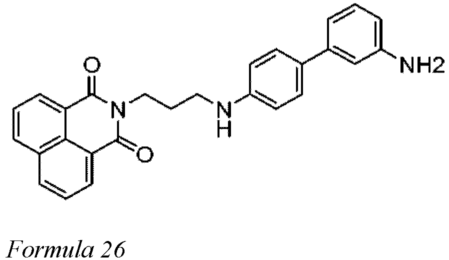

According to a preferred embodiment of the invention the molecule of Formula I is Formula 27;

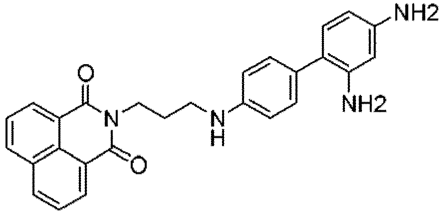

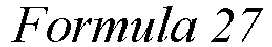

According to a preferred embodiment of the invention the molecule of Formula I is Formula 28;

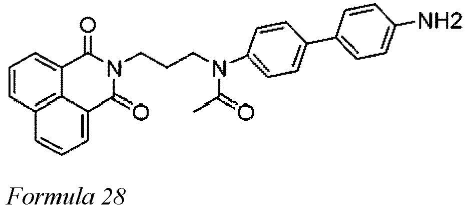

According to a preferred embodiment of the invention the molecule of Formula I is Formula 29;

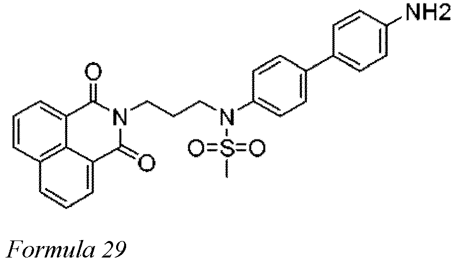

According to a preferred embodiment of the invention the molecule of Formula I is Formula 30;

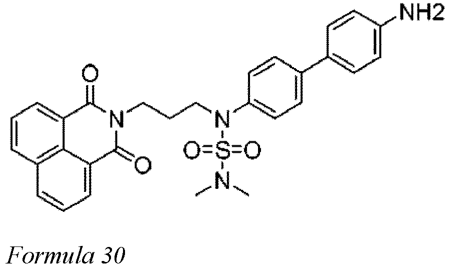

According to a preferred embodiment of the invention the molecule of Formula I is Formula 31;

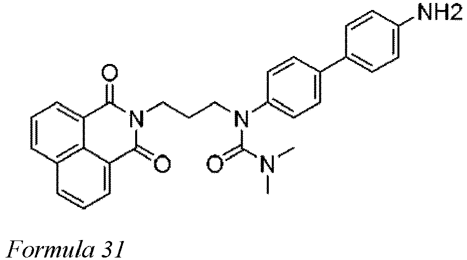

According to a preferred embodiment of the invention the molecule of Formula I is Formula 32;

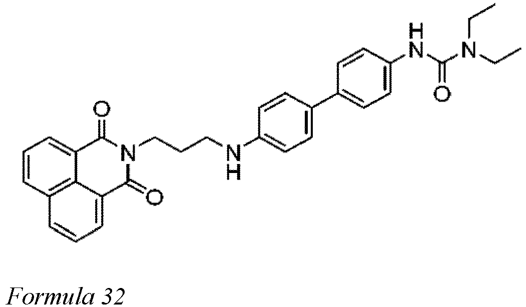

According to a preferred embodiment of the invention the molecule of Formula I is Formula 33;

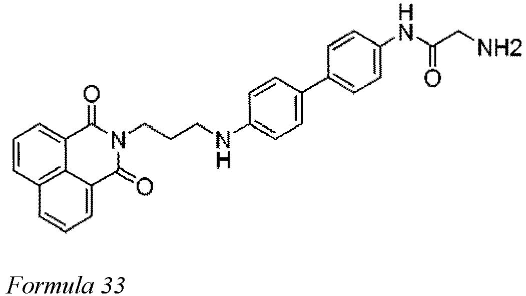

According to a preferred embodiment of the invention the molecule of Formula I is Formula 34;

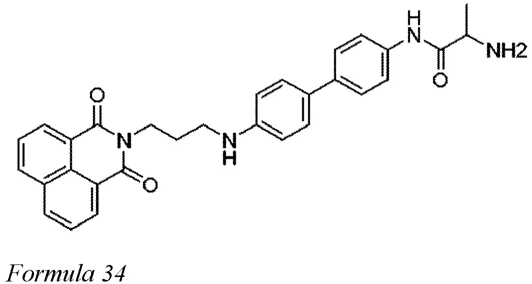

According to a preferred embodiment of the invention the molecule of Formula I is Formula 35;

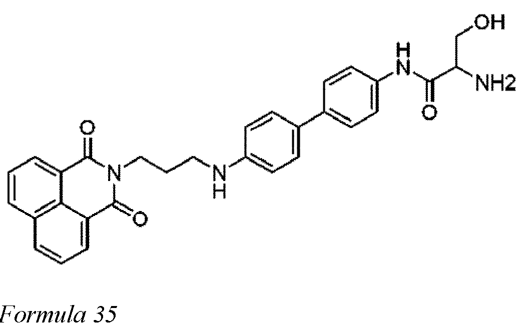

According to a preferred embodiment of the invention the molecule of Formula I is Formula 36;

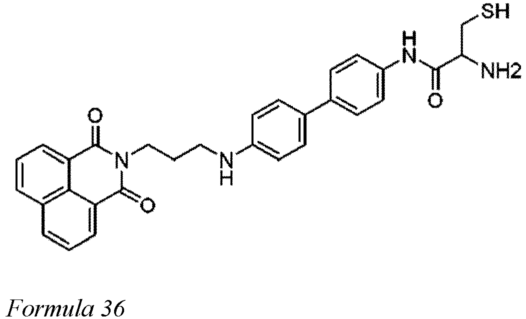

According to a preferred embodiment of the invention the molecule of Formula I is Formula 37;

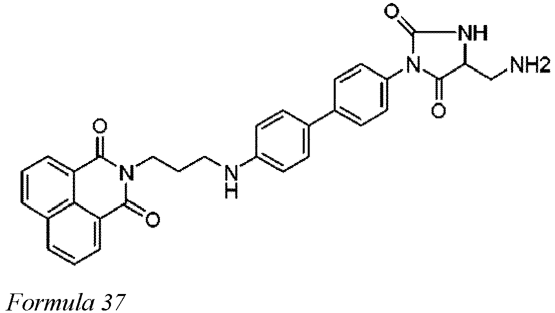

According to a preferred embodiment of the invention the molecule of Formula I is Formula 38;

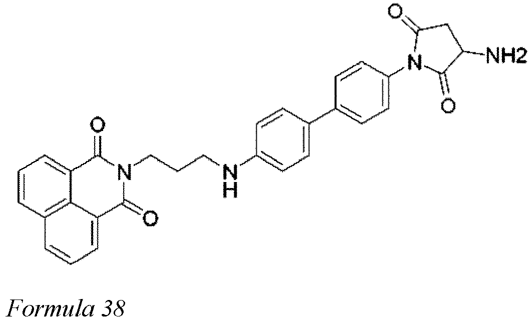

According to a preferred embodiment of the invention the molecule of Formula I is Formula 39;

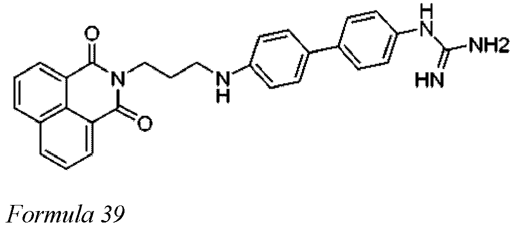

According to a preferred embodiment of the invention the molecule of Formula I is Formula 40;

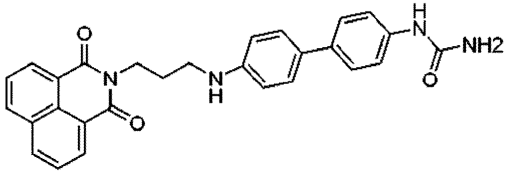

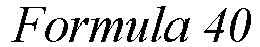

According to a preferred embodiment of the invention the molecule of Formula I is Formula 41;

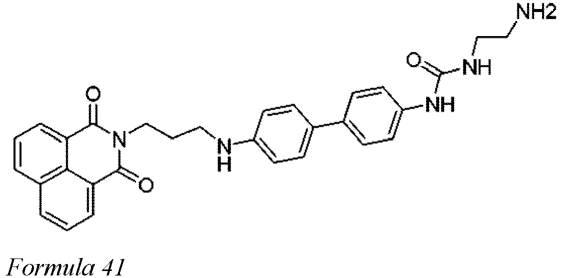

According to a preferred embodiment of the invention the molecule of Formula I is Formula 42;

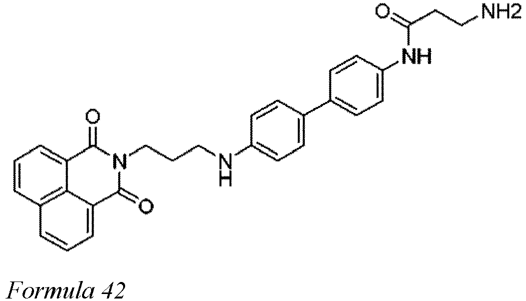

According to a preferred embodiment of the invention the molecule of Formula I is Formula 43;

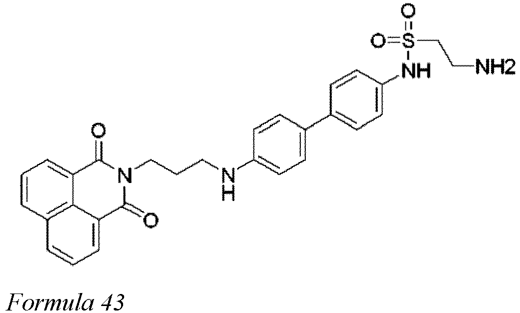

According to a preferred embodiment of the invention the molecule of Formula I is Formula 44;

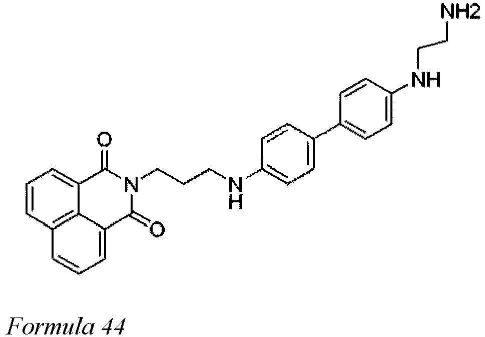

According to a preferred embodiment of the invention the molecule of Formula I is Formula 45;

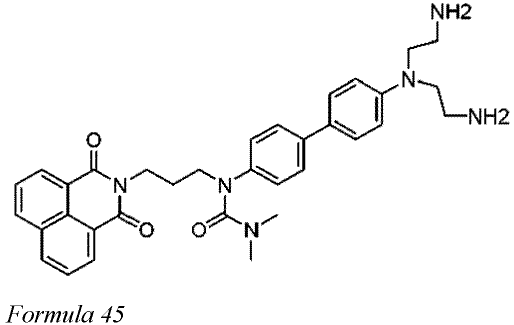

According to a preferred embodiment of the invention the molecule of Formula I is Formula 46;

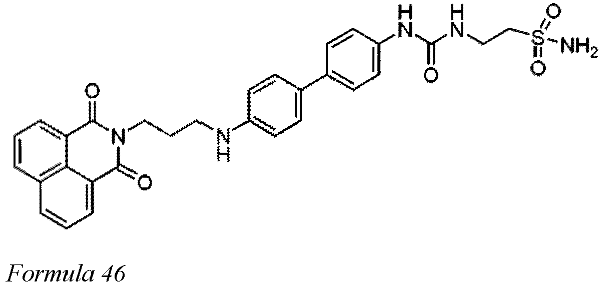

According to a preferred embodiment of the invention the molecule of Formula I is Formula 47;

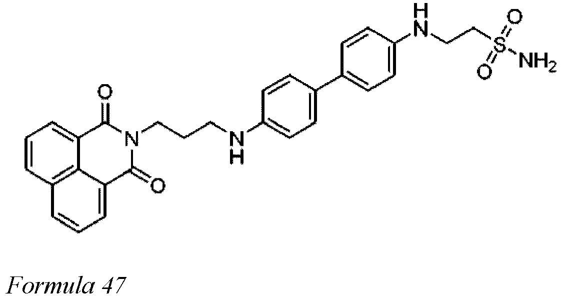

According to a preferred embodiment of the invention the molecule of Formula II is Formula 48;

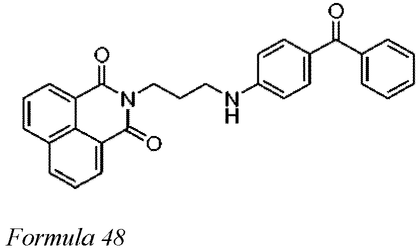

According to a preferred embodiment of the invention the molecule of Formula II is Formula 49;

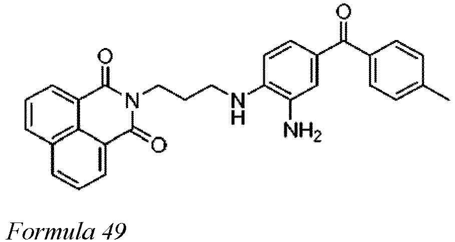

According to a preferred embodiment of the invention the molecule of Formula II is Formula 50;

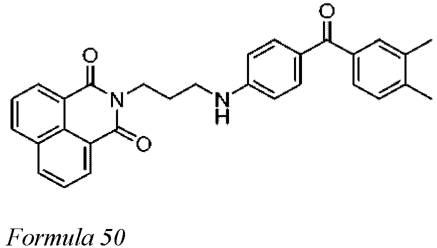

According to a preferred embodiment of the invention the molecule of Formula II is Formula 51;

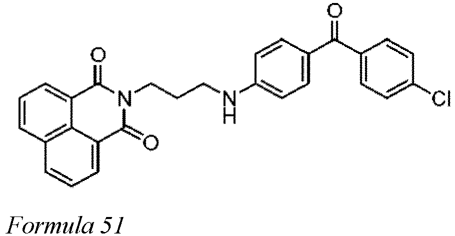

According to a preferred embodiment of the invention the molecule of Formula II is Formula 52;

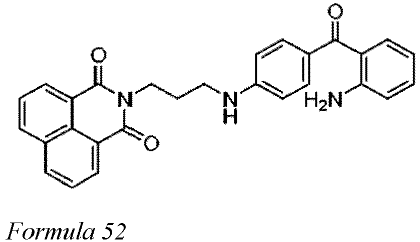

According to a preferred embodiment of the invention the molecule of Formula II is Formula 53;

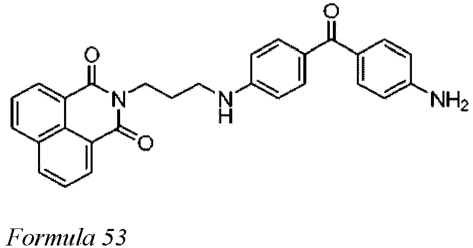

According to a preferred embodiment of the invention the molecule of Formula II is Formula 54;

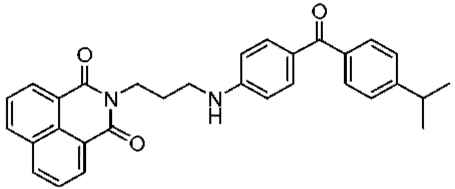

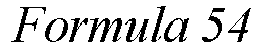

According to a preferred embodiment of the invention the molecule of Formula III is Formula 55;

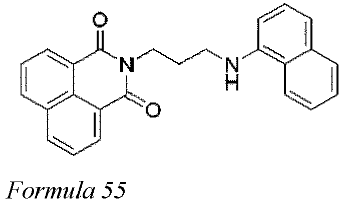

According to a preferred embodiment of the invention the molecule of Formula III is Formula 56;

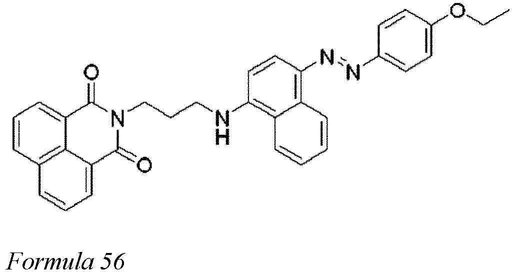

According to a preferred embodiment of the invention the molecule of Formula III is Formula 57;

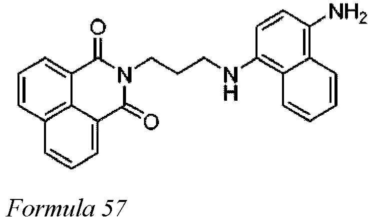

According to a preferred embodiment of the invention the molecule of Formula III is Formula 58;

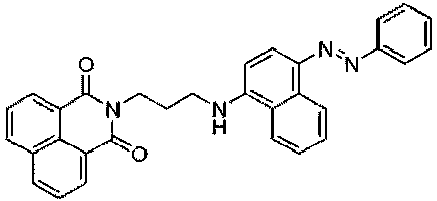

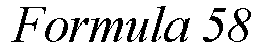

According to a preferred embodiment of the invention the molecule of Formula III is Formula 59;

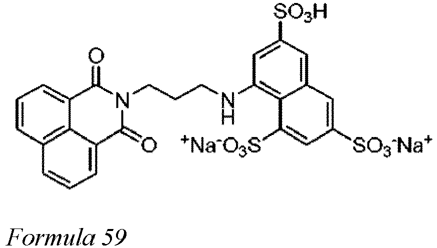

According to a preferred embodiment of the invention the molecule of Formula III is Formula 60;

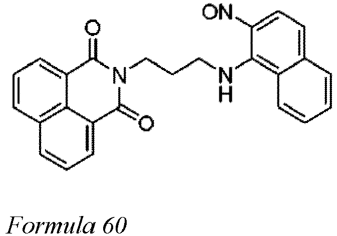

According to a preferred embodiment of the invention the molecule of Formula III is Formula 61;

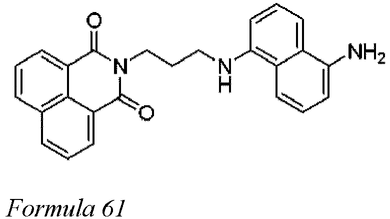

According to a preferred embodiment of the invention the molecule of Formula III is Formula 62;

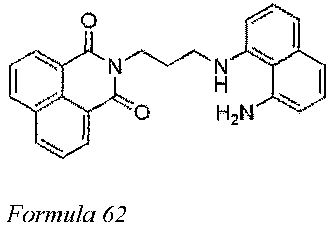

According to a preferred embodiment of the invention the molecule of Formula IV is Formula 63;

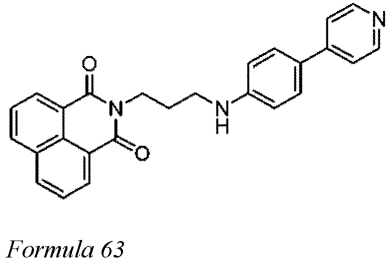

According to a preferred embodiment of the invention the molecule of Formula IV is Formula 64;

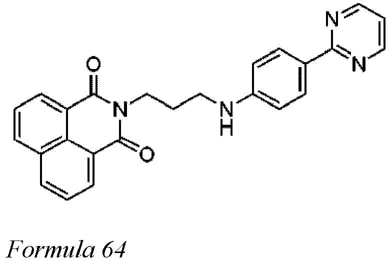

According to a preferred embodiment of the invention the molecule of Formula IV is Formula 65;

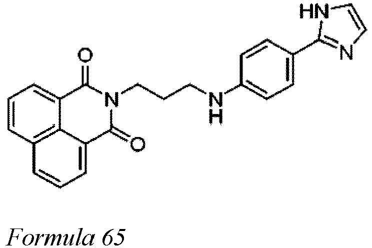

According to a preferred embodiment of the invention the molecule of Formula IV is Formula 66;

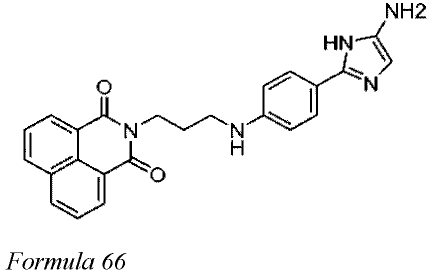

According to a preferred embodiment of the invention the molecule of Formula IV is Formula 67;

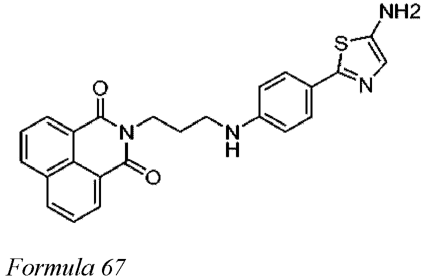

According to a preferred embodiment of the invention the molecule of Formula IV is Formula 68;

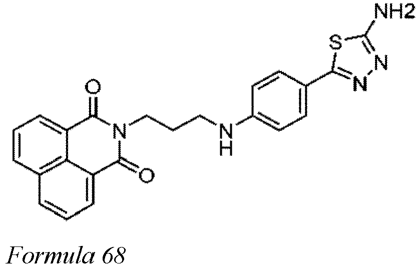

According to a preferred embodiment of the invention the molecule of Formula IV is Formula 69;

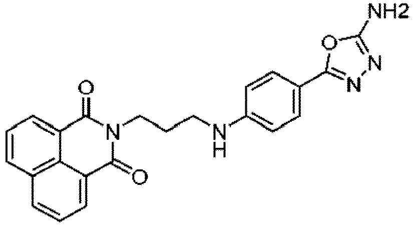

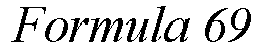

According to a preferred embodiment of the invention the molecule of Formula IV is Formula 70;

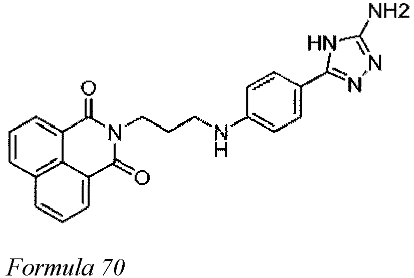

According to a preferred embodiment of the invention the molecule of Formula IV is Formula 71;

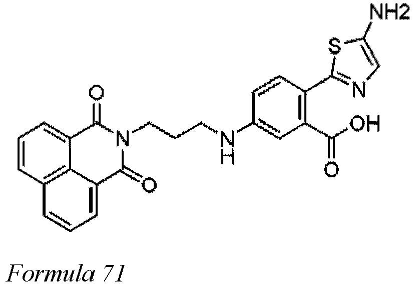

According to a preferred embodiment of the invention the molecule of Formula IV is Formula 72;

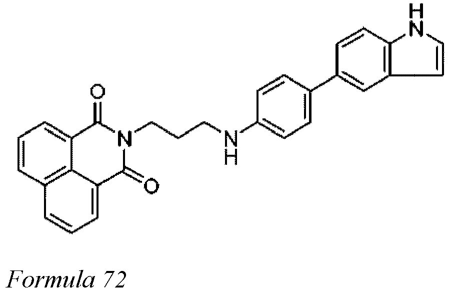

According to a preferred embodiment of the invention the molecule of Formula IV is Formula 73;

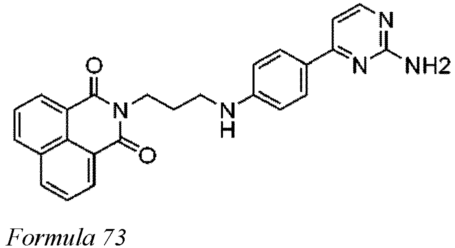

According to a preferred embodiment of the invention the molecule of Formula IV is Formula 74;

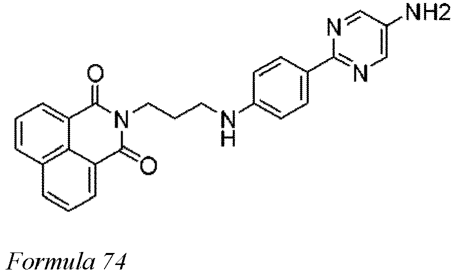

According to a preferred embodiment of the invention the molecule of Formula IV is Formula 75;

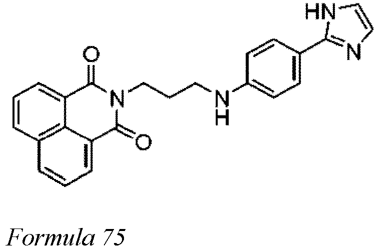

According to a preferred embodiment of the invention the molecule of Formula IV is Formula 76;

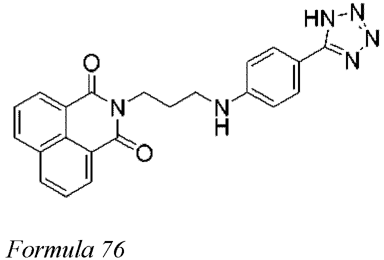

According to a preferred embodiment of the invention the molecule of Formula IV is Formula 77;

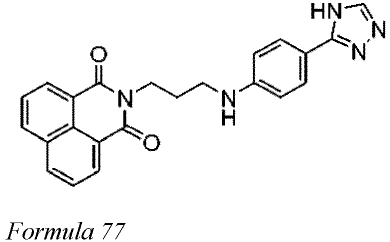

According to a preferred embodiment of the invention the molecule of Formula IV is Formula 78;

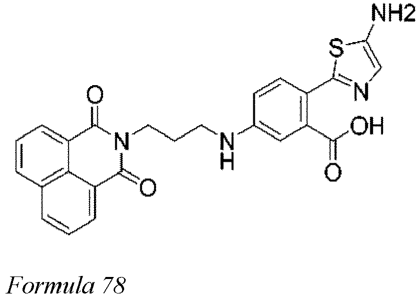

According to a preferred embodiment of the invention the molecule of Formula IV is Formula 79;

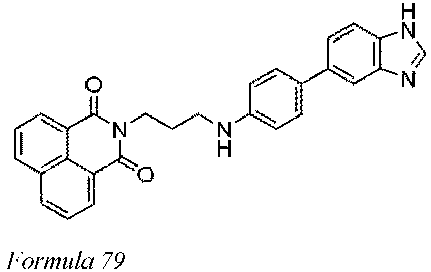

According to a preferred embodiment of the invention the molecule of Formula IV is Formula 80;

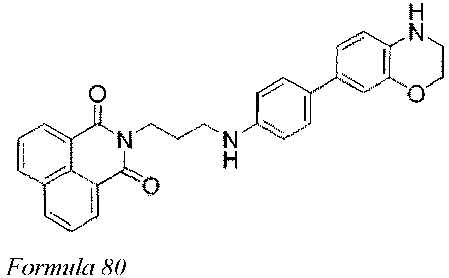

According to a preferred embodiment of the invention the molecule of Formula IV is Formula 81;

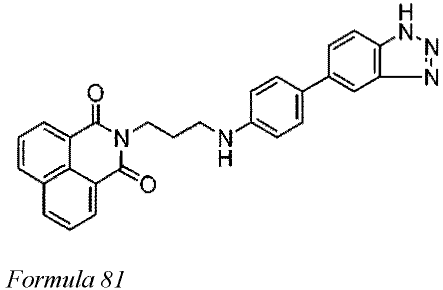

According to a preferred embodiment of the invention the molecule of Formula IV is Formula 82;

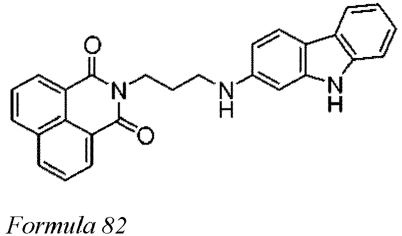

According to a preferred embodiment of the invention the molecule of Formula IV is Formula 83;

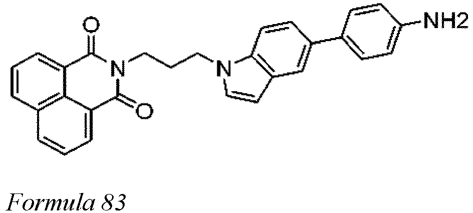

According to a preferred embodiment of the invention the molecule of Formula IV is Formula 84;

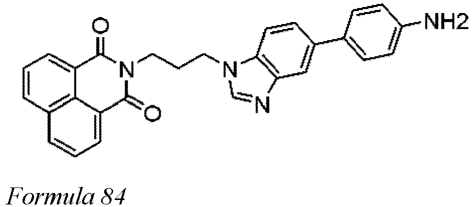

According to a preferred embodiment of the invention the molecule of Formula IV is Formula 85;

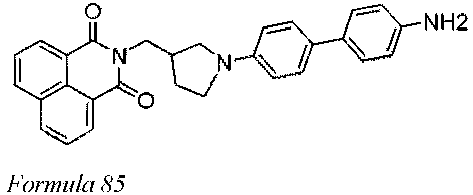

According to a preferred embodiment of the invention the molecule of Formula IV is Formula 86;

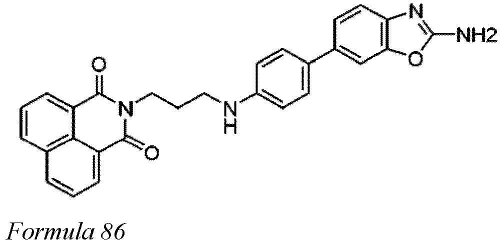

According to a preferred embodiment of the invention the molecule of Formula IV is Formula 87;

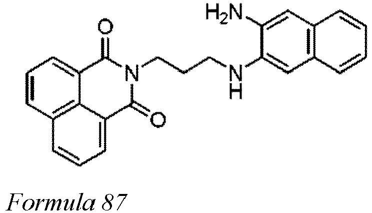

According to a preferred embodiment of the invention the molecule of Formula IV is Formula 88;

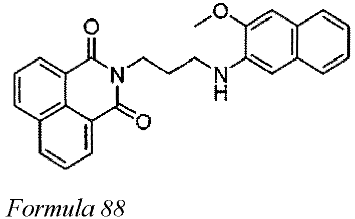

According to a preferred embodiment of the invention the molecule of Formula IV is Formula 89;

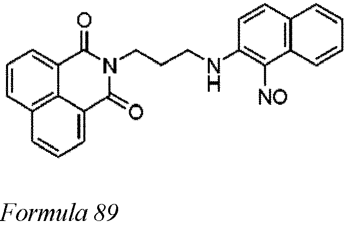

According to a preferred embodiment of the invention the molecule of Formula IV is

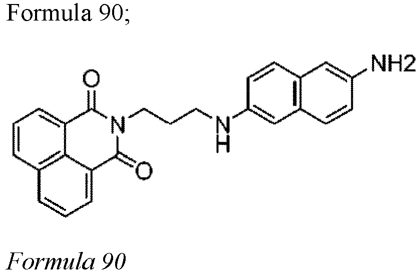

According to a preferred embodiment of the invention the molecule of Formula IV is Formula 91;

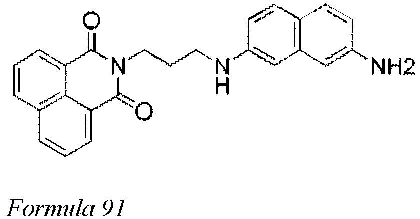

According to a preferred embodiment of the invention the molecule of Formula IV is Formula 92;

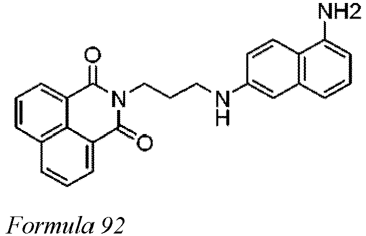

According to a preferred embodiment of the invention the molecule of Formula IV is Formula 93;

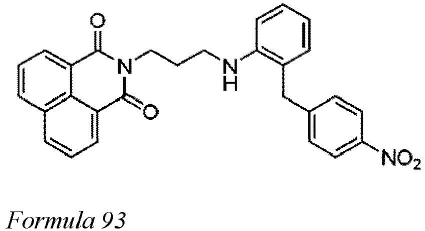

According to a preferred embodiment of the invention the molecule of Formula IV is Formula 94;

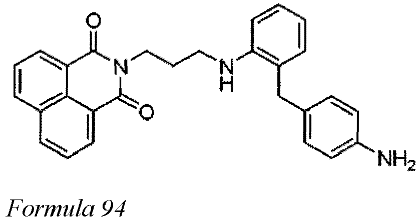

According to a preferred embodiment of the invention the molecule of Formula VII is Formula 95;

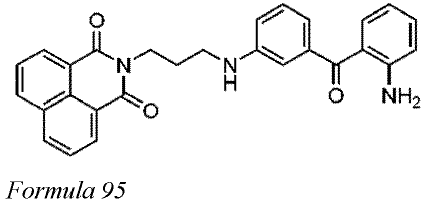

According to a preferred embodiment of the invention the molecule of Formula VII is Formula 96;

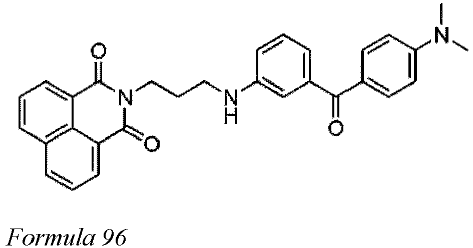

According to a preferred embodiment of the invention the molecule of Formula VII is Formula 97;

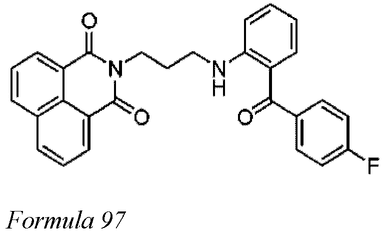

According to a preferred embodiment of the invention the molecule of Formula VII is Formula 98;

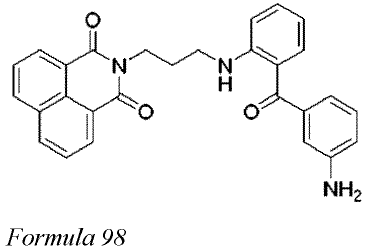

According to a preferred embodiment of the invention the molecule of Formula VII is Formula 99;

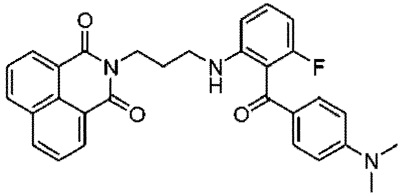

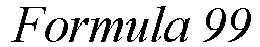

The molecules of the invention shown with formulas 1-99 whose chemical structures and specific chemical names have been given above, have been provided to further describe the invention, wherein the scope of the invention is not limited to these molecules.

The method that is used in obtaining the moelcules of Formula I, Formula II, Formula III, Formula IV, Formula V, Formula VI, Formula VII, Formula VIII comprises the steps of;
a) Reacting the agent with paramethyl sulphonyl chloride in the presence of a solvent, preferably in the presence of dichloromethane as shown with Formula X, 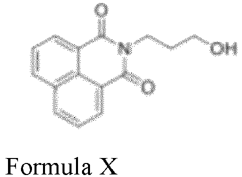
b) Mixing the obtained intermediary product (Formula Y) with NaI in the presence of a solvent, preferably acetonitrile and adding a diamino compound and K₂CO₃ solution in a solvent, preferably in acetonitrile 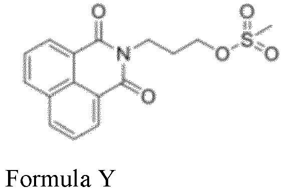
c) isolating the end product from the reaction mixture.

The term "solvent" as used herein, expresses any kind of organic solvent that can dissolve reactive agents used during the reaction process.

Said organic solvent can be selected from the group of, acetic acid, acetone, acetonitrile, benzene, 1-butanol, 2-butanol, 2-butanone, t-butyl alcohol, carbon tetrachloride, chlorobenzene, chloroform, cyclohexane, 1,2-dichloroethane, di ethylene glycol, diethylether, di ethylene glycol dimethyl ether, 1,2-dimethoxyethane (DME), dimethylformamide (DMF), dimethylsulphoxide (DMSO), 1,4-dioxane, ethanol, ethyl acetate, ethylene glycol, glycerine, heptane, hexamethylphosphoramide (HMPA), hexamethylphosphorus triamide (HMPT), hexane, methanol, methyl-butyl ether (MTBE), methylene chloride (DCM), N-methyl-yl-2-pyrrolidone (NMP), nitromethane, pentane, petrol ether, 1-propanol, 2-propanol, pyridine, tetrahydrofuran (THF), toluene, triethylamine, water, o-xylene, m-xylene, p-xylene.

The method of Method I, can be carried out at a temperature between the range of 0-90 °C or preferably at a temperature between the ranges of 25-80 °C. Each step in the method can be carried out at a temperature that is different.

The products obtained by the methods according to the invention can be purified by methods such as filtration, centrifugation, extraction, crystallization, re-crystallization, chromatography and distillation.

The chromatography method mentioned here can be selected from column chromatography, gas chromatography, liquid chromatography, inverse phase liquid chromatography.

From another aspect, the invention described the use of compounds of Formula I, Formula II, Formula III, Formula IV, Formula V, Formula VI, Formula VII, Formula VIII as a pharmaceutical product.

According to another aspect of the invention, the molecules of Formula I, Formula II, Formula III, Formula IV, Formula V, Formula VI, Formula VII, Formula VIII can be used in treating parasitic diseases.

The "parasitic diseases" term as used herein, represents diseases that are caused or infected by a parasite.

The parasitic diseases mentioned herein, are, schistosomiasis, opisthorchiasis, clonorchiasis, dicrocoeliasis, fascioliasis, paragonimiasis, fasciolopsiasis, echinococcus, teniasis, cysticercosis, difilobotriasis, sparganosis, hymenolepiasis, dracunculiasis, onchocerciasis, filariasis, elephantiasis, mansonellosis, trichinellosis, ankilostomiasis, necatoriasis, ascaridosis, strongyloidiasis, trichuriosis, enterobiasis, anisakiasis, helmintiasis, trichostrongylus, angiostrongylus gnathostomiasis, angiostrongylus, syngamiasis, acanthocephaliasis, gongylonemiasis, metastrongylosis, thelaziasis, pediculosis, pityriasis, scabies, myiasis, tungiasis, trombiculosis, scrabiasis, hirudiniasis, linguatulosis, porocephaliasis, leishmaniasis, trypanosomiasis, malaria caused by plasmodium falciparum, malaria caused by plasmodium vivax, malaria caused by plasmodium malariae, chagas disease, toxoplasma, pneumocystosis, babesiosis, piroplasmosis, acanthamoebiasis, naegleriasis and/or microsporidiosis.

According to a preferred embodiment of the invention, the molecules of Formula I, Formula II, Formula III, Formula IV, Formula V, Formula VI, Formula VII, Formula VIII can be used in a drug suitable to be used in treating leishmaniasis.

The invention preferably is related to treating leishmaniasis with the molecules of Formula I, Formula II, Formula III, Formula IV, Formula V, Formula VI, Formula VII, Formula VIII. The leishmaniasis disease can be cutaneous, mucocutaneous or visceral leishmaniasis disease.

From another aspect the invention is related to the use of the molecules of Formula I, Formula II, Formula III, Formula IV, Formula V, Formula VI, Formula VII, and Formula VIII to treat infectional diseases caused by parasites such as *L. Arabica, L. archibaldi, L. aristedesi, L. braziliensis, L. chagasi, L. colombiensis,L. Deanei, L. donovani, L. enrietii, L. equatorensis, L. forattinii, L. garnhami, L. gerbil, L.guyanensis, L. herreri, L. hertigi, L. infantum, L. killicki, L. lainsoni, L. major, L. Mexicana, L. naiffi, L. panamensis, L. peruviana, L. pifanoi, L. shawi, L. tarentolae, L. tropica, L. turanica, L. venezuelensis.*

According to another aspect of the invention, the molecules of Formula I, Formula II, Formula III, Formula IV, Formula V, Formula VI, Formula VII, Formula VIII are used in treating bacterial diseases.

The "bacterial diseases" term as used herein, represents diseases arising from infections that are caused by pathogenic bacteria.

The bacterial diseases within the scope of the invention can be any bacterial disease formed of bacteria belonging to the main group of bacilli, Bartonella, bordetella, borrelia, brucella, campylobacter, chlamydia, chlamydophila, clostridium, corynebacterium, enterococcus, eccheria, Francisella, Haemophilus, helicobacter, legionella, leptospira, listeria, mycobacterium, mycoplasma, Neisseria, pseudomonas, rickettsia, salmonella, shigella, staphylococcus, streptococcus, treponema, ureaplasma, vibrio, yersinia.

Said bacterial diseases are bubonic fever, pneumonic fever, cholera, syphilis, congenital syphilis, post streptotoxicglomerulonephritis, puerperal fever, impetigo, erysipelas, rheumatic fever, scarlet fever, streptococcall pharyngitis, sepsis, acute bacterial pneumonia, menengitis, otitis media, endometritis, neonatal sepsis, neonatal menengitis, neonatal pneumonia, cystitis, osteomyelitis, toxic shock syndrome, staphylococcal food poisoning, shigellose, salmonella, paratyphoid, typhoid, rocky mountain spotted fever, urinary tract infections, cornea infections, pneumonia, endocarditis, menengitis, gonorrhea, pelvic inflammatory disease, tuberculosis, leprosy, listeriosis, leptospirosis, legionary disease, peptic ulcer, upper respiratory tract infections, chronic gastritis, bronchitis, septic arthritis, tularemia, bloody diarrhea, urinary tract infections, endocarditis, biliary tract infections, diphtheria, tetanus, parrot disease, trachoma, neonatal conjunctivitis, urethritis, epididymitis, prostatitis, lymphogranulomatosis, atypical pneumonia, Guillain-Barre Syndrome, brucellosis, Lyme disease.

According to another aspect of the invention, the molecules of Formula I, Formula II, Formula III, Formula IV, Formula V, Formula VI, Formula VII, Formula VIII are used in treating viral diseases.

The term "viral diseases" used within the scope of the present invention defines all of the diseases formed by the entrance of pathogenic viruses and infective virus particles (virions) into cells after bonding themselves to cells.

The viral diseases mentioned can be a disease caused by any kind of virus belonging to the main families of adenoviridae, herpesviridae, papillomaviridae, polyomaviridae, poxviridae, heradnaviridae, parvoviridae, astroviridae, caliciviridae, picomaviridae, coronaviridae, flaviviridae, togaviridae, hepeviridae, retroviridae, ortomiksoviridae, arenaviridae, bunyaviridae, filoviridae, paramiksoviridae, rabdoviridae, reoviridae.

Specific examples belonging to said viral diseases are gastroenteritis, keratoconjunctivitis, pharyngitis, croup, pneumonia, cystitis, foot and mouth disease, pleurodnia, septic meningitis, pericarditis, myocarditis, Burkitt's Lymphoma, Hodgkin's lymphoma, nasopharyngeal carcinoma, acute hepatitis, chronic hepatitis, hepatic cirrhosis, hepatocellular carcinoma, tonsillitis, cytomegalic inclusion disease, Kaposi's sarcoma, Castleman disease, influenza, measles, Reye's syndrome, mumps, anogenital warts, poliomyelitis, rabies, congenital rubella, rubella, smallpox, zoster, congenital smallpox.

According to another aspect of the invention, the molecules of Formula I, Formula II, Formula III, Formula IV, Formula V, Formula VI, Formula VII, Formula VIII are used in treating neoplastic diseases.

The term "neoplastic diseases" used within the scope of the invention related to a physiological condition, for example, cancer, which is characterized by malign tumors or uncontrolled cell growth. The terms "neoplastic diseases" and "cancer" are used interchangeably with each other. Cancer examples although not limited to these, comprise carcinomas, lymphomas, blastoma sarcomas, and leukemia.

Carcinoma, as used herein, expresses a type of cancer formed of epithelium cells.

Lymphoma, as used herein, describes a cancer type developed from lymphocytes.

Blastoma, as used herein, describes a cancer type progressing from precursor cells also known as blast cells.

Sarcoma, as used herein, refers to the type of cancer resulting from altered cells of mesenchymal origin.

Leukemia, as used herein, expresses the type of cancer that is induced in the bone marrow and causes the formation of the high number of abnormal white blood cells.

Specific examples related to cancer types comprises breast cancer, prostate cancer, colorectal cancer, skin cancer, small cell lung cancer, non-small cell lung cancer, mesothelioma, gastrointestinal cancer, pancreatic cancer, glioblastoma, vulva cancer, cervical cancer, endometrial carcinoma, ovarian cancer, liver cancer, hepatoma, bladder cancer, kidney cancer, salivary gland carcinoma, thyroid cancer and various head and neck cancers.

The invention is furthermore related to a pharmaceutical composition comprising the compounds of Formula I, Formula II, Formula III, Formula IV, Formula V, Formula VI, Formula VII, Formula VIII of the invention.

Said pharmaceutical compositions can comprise at least another active agent in addition to the compounds of Formula I, Formula II, Formula III, Formula IV, Formula V, Formula VI, Formula VII, Formula VIII.

According to another embodiment of the invention the compounds of Formula I, Formula II, Formula III, Formula IV, Formula V, Formula VI, Formula VII, Formula VIII can be used together with other active agents which have antiparasitic, antibacterial, antiviral, antineoplastic and/or cytotoxic and/or antimetastatic effects or with combinations comprising two/three of these agents. The second active agent can not only be formulated together with the compounds of Formula I, Formula II, Formula III, Formula IV, Formula V, Formula VI, Formula VII, Formula VIII, but it can also be sold in packages suitable to be used together after being formulated separately from the compounds of Formula I, Formula II, Formula III, Formula IV, Formula V, Formula VI, Formula VII, Formula VIII.

According to another preferred embodiment of the invention, the compounds of Formula I, Formula II, Formula III, Formula IV, Formula V, Formula VI, Formula VII, Formula VIII is combined with at least one active agent which has antibacterial effects.

The active compounds which have antibacterial effects that is used in combination with the compounds of Formula I, Formula II, Formula III, Formula IV, Formula V, Formula VI, Formula VII, Formula VIII, are selected from the group of amikacin, gentamicin, kanamycin, neomycin, netilmicin, tobramycin, paromycin, streptomycin, spectinomycin, geldanamycin, herbimycin, rifaximin, lorcarbef, ertapenem, doripenem, imipenem, meropenem, cefadroxil, cefazolin, cefalotin, cefalexin, cefaclor, cefamandol, cefoxitin, cefprozil, cefuroxime, cefixime, cefdinir, cefditoren, cefoperazone, cefotaxime, cefpodoxime, ceftazidime, ceftibutene, ceftizoxime, cefepime, ceftaroline fosamil, ceftobiprole, teicoplanin, vancomycine, televancin, dalbavancin, oritavancin, clindamycin, lincomycin, daptomycin, azithromycin, clarithromycin, dirithromycin, erythromycin, roxithromycin, troleandomycin, telithromycin, spiramycin, aztreonam, furazolidone, nitrofurantoin, linezolid, posizolid, radezolid, torezolid, amoxicillin, ampicillin, azlocillin, carbenicillin, cloxacillin, dicloxacillin, flucloxacillin, mezlocillin, meticillin, nafcillin, oxacillin, penicillin G, penicillin V, piperacillin, temocillin, ticarcillin, clavulanate, sulbactam, tazobactam, bacitracin, colistin, polymyxin B, ciprofloxacin, enoxacin, gatifloxacin, gemifloxacin, levofloxacin, lomefloxacin, moxifloxacin, nalidixic acid, norfloxacin, ofloxacin, trovafloxacin, grepafloxacin, sparfloxacin, temafloxacin, mafenide, sulfacetamide, sulfadiazine, sulfadimethoxine, sulfamethizole, sulfamethoxazole, sulfanilamide, sulfasalazine, sulfisoxazole, trimethoprim, demeclocycline, doxycycline, minocycline, oxytetracycline, tetracycline, clofazimine, dapsone, capreomycin, cycloserine, ethambutol, isoniazid, pyrazinamide, rifampicin, rifabutin, rifapentine, streptomycin, arsphenamine, chloramphenicol, fosfomycin, fucidic acid, metronidazole, mupirocin, platensimycin, quinupristin, dalfopristin, thiamphenicol, tigecycline, tinidazole, trimethoprim or combinations comprising two or three of said agents.

According to a preferred embodiment of the invention, the compounds of Formula I, Formula II, Formula III, Formula IV, Formula V, Formula VI, Formula VII, Formula VIII is combined with at least one active agent which has antiviral effects.

The active agents that have antiviral effects, which can be used in combination with the compounds of Formula I, Formula II, Formula III, Formula IV, Formula V, Formula VI, Formula VII, Formula VIII is selected from the group of abakacir, acyclovir, adefovir, amantadine, amprenavir, ampligen, arbidol, atazanavir, atripla, balavir, cidofovir, combivir, dolutegravir, darunavir, delavirdine, didanosine, docosanol, edoxudine, efavirenz, emtricitabine, enfuvirtide, entecavir, ecoliver, famsiklovir, fomivirsen, fosamprenavir, foscarnet, fosfonate, ganciclovir, ibacitabine, imunovir, idoxuridine, imiquimod, indinavir, inosine, interpherone type I, interpherone type II, interpherone type III, interpherone, lamivudine, lopinavir, loviride, maraviroc, moroxydine, methisazone, nelfinavir, nevirapine, nexavir, nitazoxanide, norvir, oseltamivir, peginterferon alfa-2a, penciclovir, peramivir, pleconaril, podofilotoksin, protease inhibitor, nucleocyte analogues, raltegravir, ribavirin, rimantadine, ritonavir, pyramidine, saquinavir, sofosbuvir, stavudine, telaprevir, tenofovir, tipranavir, trifluridine, trizivir, tromantadine, truvada, valaciclovir, valganciclovir, vidarabine, viramidine, zalcitabine, zanamivir, zidovudine or from combinations comprising two or three of said agents.

According to a preferred embodiment of the invention, the compounds of Formula I, Formula II, Formula III, Formula IV, Formula V, Formula VI, Formula VII, Formula VIII is combined with at least one active agent which has antiparasitic effects.

Active compounds that have antiparasitic effect which can be used in combination with compounds of Formula I, is selected from the group of, Nitazoxanide, melarsoprol, eflomithine, metronidazole, tinidazole, miltefosine, mebendazole, pyrantel pamoate, thiabendazole, diethylcarbamazine, ivermectin, niclosamide, praziquantel, albendazole, rifampin, amphotericin B, fumagillin, furazolidone, nifursemizone, nitazoxanide, omidazole, paromomycin sulphate, pentamidine, pyrimethamine, tinidazole, albendazole, mebendazole, thiabendazole, fenbendazole, triclabendazole, flubendazole, abamectin, diethylcarbamazine, ivermectin, suramin, pyrantel pamoate, levamisole, niclosamide, nitazoxanide, oxyclonazide, monepantel, derquantel, amphotericin B, urea stibamine, sodium stibogluconate, meglumine antimoniate, paromomycin, miltefosine, fluconazole, pentamidine or combinations comprising two or three of said agents.

According to a preferred embodiment of the invention, the compounds of Formula I, Formula II, Formula III, Formula IV, Formula V, Formula VI, Formula VII, Formula VIII is combined with at least one active agent which has antineoplastic effects.

The active compounds which provide said antineoplastic effects that can be used in combination with the compounds of Formula I, Formula II, Formula III, Formula IV, Formula V, Formula VI, Formula VII, Formula VIII, is selected from the group of cyclophosphamide, iphosphamide, temozolomide, kapecitabin, 5-fluoro uracil, methotrexate, gemcitabine, pemetrexed, mitomycin, bleomycin, epirubicin, doxorubicin, etoposide, paclitaxel, irinotecan, osdocetaxel, vincristin, opcarboplatin, cisplatin, ocaliplatin, setcabecab, dibabecab, dibabec, rituximab, sunitinib, zoledronate, abiraterone, anastrozole, bicalutamide, exemestane, goserelin, medroxyprogesterone, octreotide, tamoxifen, bendamustine, carmustine, chlorambucil, lomustine, melphalan, procarbazine, streptozotocin fludarabine, raltitrexed, actinomycin D, dactinomycin, doxorubycin, mitoxantrone, erybulin, topotecan, vinblastine, vinorelbine, afatinib, aflibersept, chrysotinib, dabrafenib, interferon, ipilimumab, lapatinib, nivolumab, panitumumab, pembrolizumab, pertuzumab, sorafenib, trastuzumab, emtansin, temsoril, vemurafenib, ibandronic acid, pamidronate, bexarotane, buserelin, Cyproterone, degareliks, folinic acid, fulvestrant, lanreotide, lenalidomide, letrozole, leuprorelin, megestrol, mesna, thalidomide or combinations comprising two or three of said agents.

According to another embodiment of the invention at least another active agent can be formulized together with or separately from the compounds of Formula I, Formula II, Formula III, Formula IV, Formula V, Formula VI, Formula VII, Formula VIII, and at least said other active agents can have the same dosage as, or can have a different dosage from the compounds of Formula I, Formula II, Formula III, Formula IV, Formula V, Formula VI, Formula VII, Formula VIII.

In the case that at least another active agent mentioned above with the compounds of Formula I, Formula II, Formula III, Formula IV, Formula V, Formula VI, Formula VII, Formula VIII, is used, the other active agents, can be given at the same time with, successively with, or sequentially with the agents of Formula I, Formula II, Formula III, Formula IV, Formula V, Formula VI, Formula VII, Formula VIII.

The formulations according to the invention can comprise at least an excipient in addition to the active agents of Formula I, Formula II, Formula III, Formula IV, Formula V, Formula VI, Formula VII, Formula VIII.

The dosage range of the active agents that can be used together with Formula I, Formula II, Formula III, Formula IV, Formula V, Formula VI, Formula VII, Formula VIII shall be determined according to the requirements of the patient, the phase of the disease and the active agent to be used. The determination of a dose according to a specific situation is known by those that are skilled in the art.

The expression "comprising" within the scope of the invention is also used to mean "encompassing".

Where it is technically suitable, the embodiments of the invention can be combined.

The invention will be further described below by referring to the examples.

### EXAMPLES:

### Example 1: 2-(3-hydroxypropyl)-1H-benzo[de]isoquinoline-1,3(2H)-dione (Formula X) synthesis method

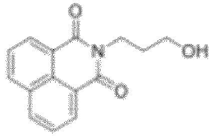

3-Amino-1-propanol (1 mmol) and DBU (1 mmol) is added into 4-amino-1,8-naphthalic anhydrite (1 mmol) solution dissolved in DMF and it is enabled to be mixed for 4 hours at 70 °C. Following this iced water is added into the medium, and the solid established is filtered and a pure product is obtained. The progression of the reaction is checked with a DCM/MeOH:40/1 solvent system by conducting a TLC study. (Yield: %98)
Analysis Data: LC-MS: m/z 256 [M+1]

### Example 2: 3-(1,3-dioxo-1H-benzo[de]isoquinoline-2(3H)-il)propyl methanesulfonate (Formula Y) synthesis method

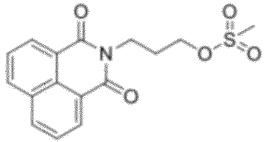

2-(3-hydroxypropyl)-1H-benzo[de]isoquinoline-1,3(2H)-dione is dissolved in DCM.

Following this TEA is added to the medium (1.2 mmol) and it was mixed for 15 minutes at 0°C. Following this para-methyl sulphonyl chloride (1.2 mmol) has been added into the medium and the reaction has been mixed for 2 hours. The progression of the reaction is checked with a DCM/MeOH (10/1) solvent. The reaction process was stopped with precipitation in ice water and filtering, and a pure product was obtained. (Yield: %86)
Analysis Data: LC-MS: m/z 334 [M+1]

### Example 3: Synthesis method of Formula 1

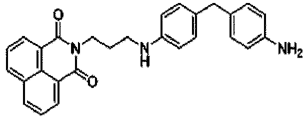

Formula Y (1 mmol) was mixed with NaI (6 mmol) for 2 hours at 70°C in ACN medium. At the same time, it is enabled for Diaminodiphenylmethane (1 mmol) and K₂CO₃ (6 mmol) to be mixed in ACN medium at 70°C for 2 hours in a reaction flask. Following this, these two reactions were mixed with each other and the reaction process was continued. TLC control was conducted and at the end of 12 hours, the reaction solvent was removed in a vacuum system. Following this extraction was carried out with aqueous NaCl solution and chloroform and the reaction was ended. The organic phase was placed inside dry Na₂SO₄ and was filtered and the solvent was removed. The purification process was carried out with column chromatography using a DCM / MeOH (40: 1) solvent system during the purification of the product.
Analysis Data: LC-MS: m/z 436 [M+1].
¹H NMR (500 MHz, CDCl3): δ= 1.95 (p, *J* = 6.7 Hz, 2H, CH₂), 3.10 (t, *J* = 6.5 Hz, 2H, NHCH₂), 3.66 (s, 2H, PhCH₂), 4.20 (t, *J* = 6.9 Hz, 2H, NCH₂), 6.52 - 6.48 (m, 4H, 4xArH), 6.87 (dd, *J* = 8.2, 5.3 Hz, 4H, 4xArH), 7.66 - 7.61 (m, 2H, 2xArH), 8.09 (dd, *J* = 8.3, 0.7 Hz, 2H, 2xArH), 8.48 (dd, *J* = 7.3, 0.9 Hz, 2H, 2xArH) ppm.

### Example 4: Synthesis method of Formula 9

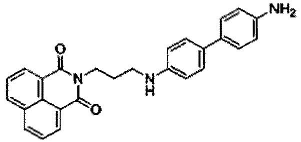

Formula Y (1 mmol) was mixed with NaI (6 mmol) for 2 hours at 70°C in ACN medium. At the same time, it is enabled for Benzidine (1 mmol) and K₂CO₃ (6 mmol) to be mixed in ACN medium at 70°C for 2 hours in a reaction flask. Following this, these two reactions were mixed with each other and the reaction process was continued. TLC control was conducted and at the end of 12 hours, the reaction solvent was removed in a vacuum system. Following this extraction was carried out with aqueous NaCl solution and chloroform and the reaction was ended. The organic phase was placed inside dry Na₂SO₄ and was filtered and the solvent was removed. The purification process was carried out with column chromatography using a DCM / MeOH (20: 1) solvent system during the purification of the product.
Analysis Data: LC-MS: m/z 423 [M+1].
¹H NMR (500 MHz, CDCl3): δ= 1.99-2.06 (m, 2H, CH₂), 3.18 (t, *J* = 6.4 Hz, 2H, NHCH₂), 4.26 (t, *J* = 6.8 Hz, 2H, NCH₂), 6.67 - 6.61 (m, 4H, 4xArH), 7.30 - 7.25 (m, 2H, 4xArH), 7.69 (dd, *J* = 12.8, 5.2 Hz, 2H, 2xArH), 8.16 (dd, *J* = 8.2, 0.7 Hz, 2H, 2xArH), 8.55 (dd, *J* = 7.3, 0.9 Hz, 2H, 2xArH) ppm.

### Example 5: 2-(3-((5-amino naphthalene-1-il)amino)propyl)-1H-benzo[de]isoquinoline-1,3(2H)-dione, (C₂₅H₂₁N₃O₂) (Formula 61) synthesis method

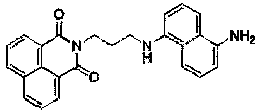

Formula Y (1 mmol) was mixed with NaI (6 mmol) for 2 hours at 70°C in ACN medium. At the same time, it is enabled for 1.5-Diaminodiphenylmethane (1 mmol) and K₂CO₃ (6 mmol) to be mixed in ACN medium at 70°C for 2 hours in a reaction flask. Following this, these two reactions were mixed with each other and the reaction process was continued. TLC control was conducted and at the end of 12 hours, the reaction solvent was removed in a vacuum system. Following this extraction was carried out with aqueous NaCl solution and chloroform and the reaction was ended. The organic phase was placed inside dry Na₂SO₄ and was filtered and the solvent was removed. The purification process was carried out with column chromatography using a DCM / MeOH (25: 1) solvent system during the purification of the product.
Analysis Data: LC-MS: m/z 396 [M+1].
¹H NMR (500 MHz, CDCl3): δ= 2.13 (p, *J* = 6.6 Hz, 2H, CH₂), 3.33 (t, *J* = 6.3 Hz, 2H, NHCH₂), 4.33 (t, *J* = 6.7 Hz, 2H, NCH₂), 6.57 (d, *J* = 7.6 Hz, 1H, ArH), 6.71 (d, *J* = 73 Hz, 1H, ArH), 7.09 (d, *J* = 8.4 Hz, 1H, ArH), 7.22 (dd, *J* = 8.8, 1.3 Hz, 1H, ArH), 7.28 - 7.24 (m, 1H, ArH), 7.41 (t, *J* = 6.1 Hz, 1H, ArH), 7.70 (dt, *J* = 11.6, 5.8 Hz, 2H, 2xArH), 8.17 (dd, *J* = 8.3, 0.9 Hz, 2H, 2xArH), 8.58 (dd, *J* = 7.3, 1.0 Hz, 2H, 2xArH) ppm.

### Example 6: 2-(3-((8-amino naphthalene-1-yl)amino)propyl)-1H-benzo[de]isoquinoline-1,3(2H)-dione, (C₂₅H₂₁N₃O₂) (Formula 62) synthesis method

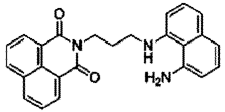

Formula Y (1 mmol) was mixed with NaI (6 mmol) for 2 hours at 70°C in ACN medium. At the same time, it is enabled for 1,8-Diaminodiphenylmethane (1 mmol) and K₂CO₃ (6 mmol) to be mixed in ACN medium at 70°C for 2 hours in a reaction flask. Following this, these two reactions were mixed with each other and the reaction process was continued. TLC control was conducted and at the end of 12 hours, the reaction solvent was removed in a vacuum system. Following this extraction was carried out with aqueous NaCl solution and chloroform and the reaction was ended. The organic phase was placed inside dry Na₂SO₄ and was filtered and the solvent was removed. The purification process was carried out with column chromatography using a DCM / MeOH (100: 1) solvent system during the purification of the product.
Analysis Data: LC-MS: m/z 396 [M+1].
¹H NMR (500 MHz, CDCl3): δ= 2.17 - 2.13 (m, 2H, CH₂), 3.19 (t, *J=* 6.5 Hz, 2H, NHCH₂), 4.34 (dd, *J=* 13.9, 6.8 Hz, 2H, NCH₂), 6.57 (t, *J* = 8.2 Hz, 1H), 6.64 (d, *J* = 7.2 Hz, 1H), 7.11 (d, *J* = 4.1 Hz, 1H), 7.13 (d*, J* = 3.7Hz, 1H, ArH), 7.18 (d, *J* = 3.3 Hz, 1H, ArH), 7.20 (d, *J* = 5.4 Hz, 1H, ArH), 7.71 - 7.67 (m, 2H, 2xArH), 8.15 (dd, *J* = 8.3, 1.0 Hz, 2H, 2xArH), 8.54 (dd, *J* = 7.3, 1.0 Hz, 2H, 2xArH) ppm.

### Example 7: Biological activity of Formula 9 against Leishmania Infantum Parasites

### Method and Materials:

Parasite Culture: Leishmania infantum (MHOM/MA/67/ITMA-P263) promastigotes are incubated in an RPMI medium (with heat-activated 10% fetal bovine serum, 2 mM L-glutamine, 20 mM HEPES, 100 U/ml penicillin, 100 µg/ml streptomycin) at 27°C. The parasites that reach the logarithmic phase (10⁶/ml) become infective.

Alamar Blue Cell Viability Test: After the Leishmania infantum (MHOM/MA/67/ITMA-P263) promastigotes were added into RPMI medium (with heat-activated 10% fetal bovine serum, 2 mM L-glutamine, 20 mM HEPES, 100 U/ml penicillin, 100 µg/ml streptomycin) at 27°C, in 96 well culture plates (Corning Glasswork, Corning, NY) such that they are 8×10⁶ parasite/wells, on the 3rd day the viability levels of parasites were measured. Alamar Blue test (Sigma-Aldrich, Germany) is applied to determine the viabilities of parasites. 10µl Alamar Blue solution is added onto the cells inside the 100µl growth medium and the cells are incubated for a day in the dark. Following the incubation period, absorbance measurement was taken with an ELISA plaque reader (Biotek, Winooski, VT) at 530/590 nm wavelength and viability analysis was observed.

### Result:

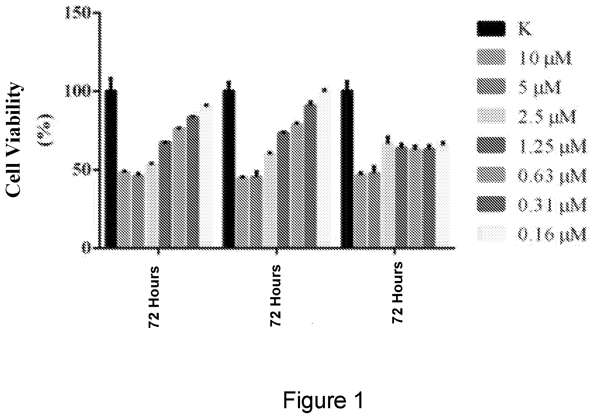

The effect of the molecule with Formula 9 defined within the scope of the invention, on *Leishmania infantum* parasites has been shown. The Formula 9 compound was added onto the parasites, and when viability was analyzed for 3 days, the death rate up to an average of 45% was observed (Figure 1). Under the light of the assays carried out, it has been determined that the developed molecule was effective on Leishmania infantum parasites.

## Claims

1. A Bisnaphthalimidopropyl (BNIP) derivative of formula I, II, III, IV, V, VI, VII or VIII;
a) Formula I; 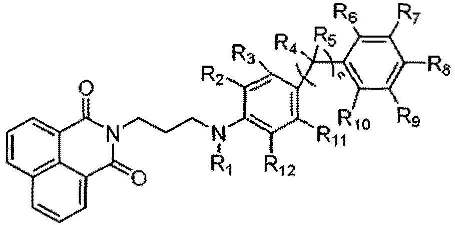
b) Formula II; 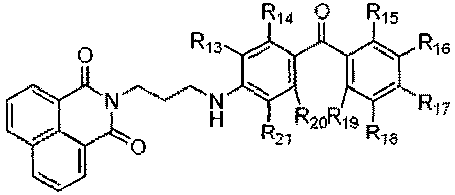
c) Formula III; 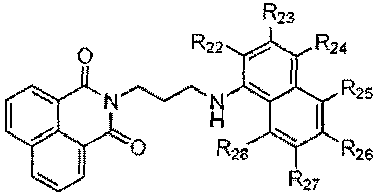
d) Formula IV; 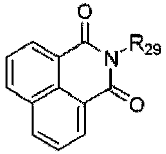
e) Formula V; 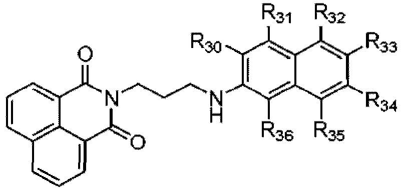
f) Formula VI; 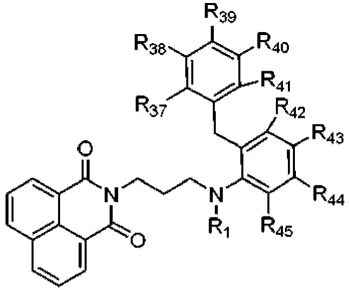
g) Formula VII; 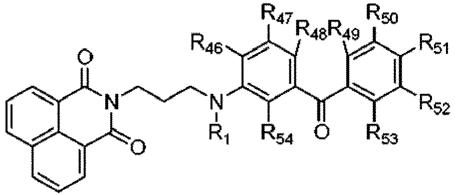
h) Formula VIII; 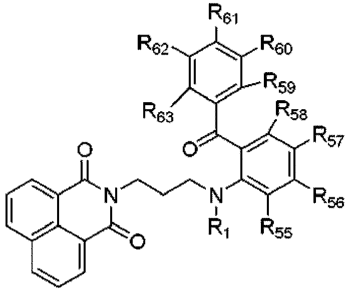 wherein;
-n is a natural number between 0 to 12,
- R₁, R₂, R₃, R₄, R₅, R₆, R₇, R₈, R₉, R₁₀, R₁₁, R₁₂, R₃₇, R₃₈, R₃₉, R₄₀, R₄₁, R₄₂, R₄₃, R₄₄, R₄₅, R₄₆, R₄₇, R₄₈, R₄₉, R₅₀, R₅₁, R₅₂, R₅₃, R₅₄, R₅₅, R₅₆, R₅₇, R₅₈, R₅₉, R₆₀, R₆₁, R₆₂, R₆₃, are independently selected from the group consisting of;
-H, -NH₂, -NHR_{X}, -NR_{X}R_{Y}, -NHNR_{X}, -N(R_{X})(NHR_{Y}), -N(R_{X})(NR_{Y}R_{Z}), R_{X}, R_{Y}, RZ, Cl, F, Br, I, -OH, -NO, -NO₂, -COOH, -OR_{X}, -SO₂R_{X}, -SO₂NHR_{X}, -SO₂NR_{X}R_{Y}, -CN, -COOR_{X}, - CONHR_{X}, -CONR_{X}R_{Y}, -NHCOCH₂R_{X}, -NHCOCHR_{X}R_{Y}, -NHCNHR_{X}, -NHCOR_{X}, - NHCONHRx, -NHSO₂R_{X};
- R₁₃, R₁₄, R₁₅, R₁₆, R₁₇, R₁₈, R₁₉, R₂₀, R₂₁ are independently selected from the group consisting of;
-H, -Cl, -F, -Br, -I, R_{X}, -NH₂, -NHR_{X}, -NR_{X}R_{Y}, -OH, -NO, -NO₂, -COOH, -OR_{X}, -COOR_{X}, - CN;
- R₂₂, R₂₃, R₂₄, R₂₅, R₂₆, R₂₇, R₂₈ are independently selected from the group consisting of -H, -Cl, -F, -Br, -I, R_{X}, -NH₂, -NHR_{X}, -NR_{X}R_{Y}, -OH, -COOH, -OR_{X}, -COOR_{X}, -CN, - SO₃H, -SO₃ Na⁺, 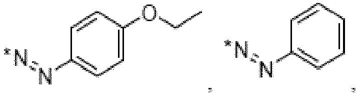 , -NO;
-R₂₉ is selected from the group consisting of; 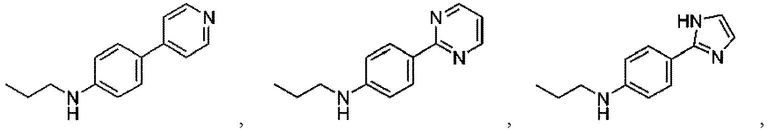 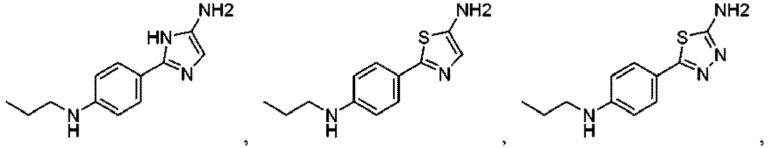 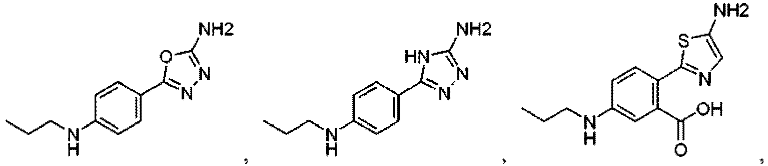 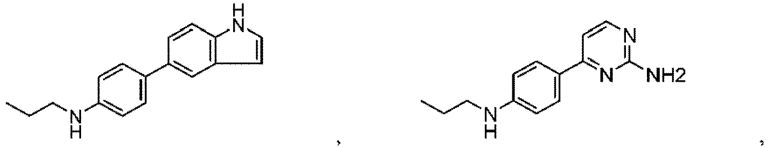 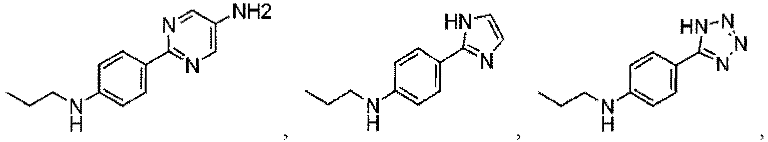 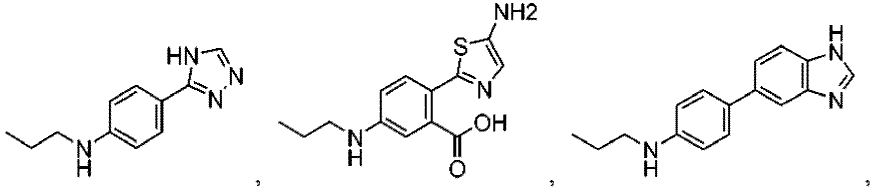 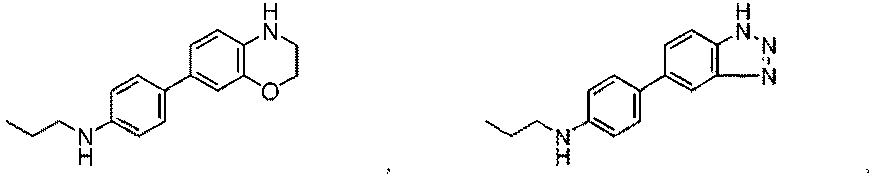 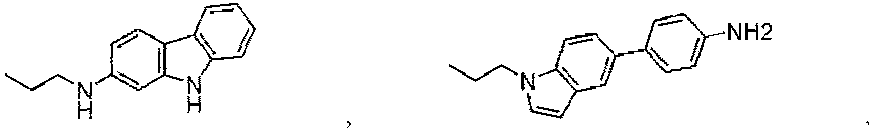 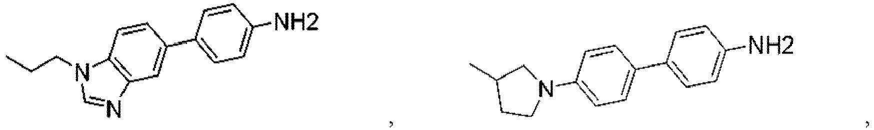 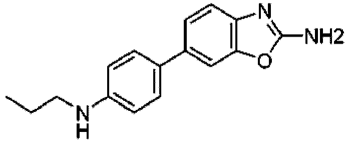
- R₃₀, R₃₁, R₃₂, R₃₃, R₃₄, R₃₅, R₃₆ are independently selected from the group consisting of; -H, -Cl, -F, -Br, -I, R_{X}, -NH₂, -NHR_{X}, -NR_{X}R_{Y}, -OH, -NO, -COOH, -OR_{X}, -COOR_{X}, -CN;
R_{X}, R_{Y}, and R_{Z} are independently selected from substituted or unsubstituted C1-C12 alkane, substituted or unsubstituted C1-C12 alkene, substituted or unsubstituted C1-C12 alkyne, substituted or unsubstituted C1-C12 primary amine, substituted or unsubstituted C1-C12 secondary amine, substituted or unsubstituted alcohol, substituted or unsubstituted alkyl cyanide, substituted or unsubstituted sulphonamide, substituted or unsubstituted C1-C12 alkyl sulphonyl, substituted or unsubstituted C3-C8 cycloalkyl, substituted or unsubstituted aryl, substituted or unsubstituted heteroaryl, optionally and independent from each other from a group formed of substituted or unsubstituted heterocyclic or heteroaromatic rings that comprise one or more heteroatoms selected from O, S and N.

2. Bisnaphthalimidopropyl (BNIP) derivative of formula I, II, III, IV, V, VI, VII or VIII according to Claim 1,
wherein n takes the value of 0 or 1 or 2 or 3 or 4 or 5 or 6 or 7 or 8 or 9 or 10 or 11 or 12 in the molecules of Formula I;
R₁, R₂, R₃, R₄, R₅, R₆, R₇, R₈, R₉, R₁₀, R₁₁ ve R₁₂ are independently selected from the group consisting of;
-H, -NH₂, -CH₃, -CH₂CH₃, -CH₂CH₂CH₃, -CH₂CH₂CH₂CH₃, -CH₂CH₂CH₂CH₂CH₃, - CH₂CH₂CH₂CH₂CH₂CH₃, CH₂CH₂CH₂CH₂CH₂CH₂CH₃, - CH₂CH₂CH₂CH₂CH₂CH₂CH₂CH₃, -CH₂CH₂CH₂CH₂CH₂CH₂CH₂CH₂CH₃, CH₂CH₂CH₂CH₂CH₂CH₂CH₂CH₂CH₂CH₃, - CH₂CH₂CH₂CH₂CH₂CH₂CH₂CH₂CH₂CH₂CH₃, - CH₂CH₂CH₂CH₂CH₂CH₂CH₂CH₂CH₂CH₂CH₂CH₃, -C(CH₃)₃, Cl, F, Br, I, -OH, - OCH₃, -OCH₂CH₃, -OCH₂CH₂CH₃, -OCH₂CH₂CH₂CH₃, -OCH₂CH₂CH₂CH₂CH₃, - OCH₂CH₂CH₂CH₂CH₂CH₃, -NHCH₃, -NHCH₂CH₃, -NHCH₂CH₂CH₃, - NHCH₂CH₂CH₂CH₃, -NHCH₂CH₂CH₂CH₂CH₃, -NHCH₂CH₂CH₂CH₂CH₂CH₃, - N(CH₃)₂, -N(CH₂CH₃)(CH₃), -N(CH₂CH₃)₂, -N(CH₂CH₃)(CH₂CH₂CH₃), - N(CH₃)(CH₂CH₂CH₃), N(CH₂CH₂CH₃)₂, -NHNH₂, -NHNHCH₃, -NHNHCH₂CH₃, - NHNHCH₂CH₂CH₃, -NHN(CH₃)₂, -NHN(CH₂CH₃)₂, -NHN(CH₃)(CH₂CH₃), - N(CH₃)(NHCH₃), -N(CH₃)(N(CH₃)₂), -COOH, -SO₂CH₃, -SO₂CH₂CH₂, - SO₂CH₂CH₂CH₃, -SO₂CH₂CH₂CH₂CH₃, -SO₂CH₂CH₂CH₂CH₂CH₃, - SO₂CH₂CH₂CH₂CH₂CH₂CH₃, -CN, -CONH₂, -CONHCH₃, -CONHCH₂CH₃, - CONHCH₂CH₂CH₃, -CONHCH₂CH₂CH₂CH₃, -CONHCH₂CH₂CH₂CH₂CH₃, - CONHCH₂CH₂CH₂CH₂CH₂CH₃, -CON(CH₃)₂, -CON(CH₃)(CH₂CH₃), - CON(CH₂CH₃)₂, -CON(CH₂CH₃)(CH₂CH₂CH₃), -CON(CH₃)(CH₂CH₂CH₃), - CON(CH₂CH₂CH₃)₂, -COCH₃, -COCH₂CH₃, -COCH₂CH₂CH₃, - COCH₂CH₂CH₂CH₃, -COCH₂CH₂CH₂CH₂CH₃, -COCH₂CH₂CH₂CH₂CH₂CH₃, - COOCH₃, -COOCH₂CH₃, -COOCH₂CH₂CH₃, -COOCH₂CH₂CH₂CH₃, - COOCH₂CH₂CH₂CH₂CH₃, -COOCH₂CH₂CH₂CH₂CH₂CH₃, -COCH(CH₃)₂, - COC(CH₃)₃, -COCH(CH₂CH₃)₂, -COCH(CH₃)(CH₂CH₃), -COCH₂CH₂CH₃, - COCH₂CH₂CH₂CH₃, -COCH₂CH₂CH₂CH₂CH₃, -COCH₂CH₂CH₂CH₂CH₂CH₃, - SO₂NH₂, SO₂NHCH₃, SO₂NHCH₂CH₃, -SO₂NHCH₂CH₂CH₃, SO₂N(CH₃)₂, - SO₂N(CH₃)(CH₂CH₃), -SO₂N(CH₂CH₃)₂, -SO₂N(CH₃)(CH₂CH₂CH₃), - SO₂N(CH₂CH₂CH₃)₂, -NHCONHCH₃, -NHCON(CH₃)₂, -NHCONHCH₂CH₃, NHCON(CH₃)(CH₂CH₃), -NHCON(CH₂CH₃)₂, -NHCOCH(CH₃)(NH₂), - NHCOCH(CH₃)₂, -NHCOCH(CH₃)(NHCH₃), -NHCOCH(CH₃)(N(CH₃)₂), - NHCOCH(CH₂CH₃)₂, -NHCOCH(CH₂OH)(NH₂), -NHCOCH(CH₂CH₂OH)(NH₂), - NHCOCH(CH₂CH₂OH)(NHCH₃), -NHCOCH(CH₂OH)(NHCH₃), NHCOCH(CH₂OH)(N(CH₃)₂), NHCOCH(CH₂OH)(N(CH₂CH₃)₂), - NHCOCH(CH₂SH)(NH₂), -NHCOCH(CH₂CH₂SH)(NH₂), - NHCOCH(CH₂CH₂CH₂SH)(NH₂), -NHCOCH(CH₂SH)(NHCH₃), - NHCOCH(CH₂SH)(NHCH₂CH₃), -NHCOCH(CH₂SH)(N(CH₃)₂), 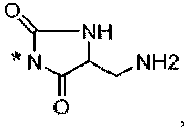 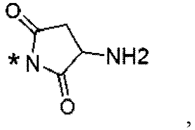 -NH(CNH)NH₂, -NH(CN(CH₃))NH₂, -NH(CN(CH₂CH₃))NH₂, - NH(CN(CH₃))NH(CH₃), -- NH(CN(CH₃))N(CH₃)₂, -NH(CNH)NHCH₃, NH(CNH)N(CH₃)₂, -NH(CO)NHCH₂CH₂NH₂, -NH(CO)NHCH₂NH₂, - NH(CO)NHCH₂CH₂NHCH₃, -NH(CO)N(CH₃)(CH₂CH₂NH₂), - NH(CO)CH₂CH₂NH₂, -NH(CO)CH₂NH₂, -NH(CO)CH₂CH₂CH₂NH₂, - NH(CO)CH₂CH₂CH₂CH₂NH₂, -NH(CO)CH₂CH₂NHCH₃, -NH(CO)CH₂CH₂N(CH₃)₂, -NH(CO)CH₂CH₂NH(CH₂CH₃), -NH(CO)CH₂CH₂N(CH₃)(CH₂CH₃), - NHSO₂CH₂CH₂NH₂, -NHSO₂CH₂CH₂CH₂NH₂, -NHSO₂CH₂CH₂NH₂HCH₃, - NHSO₂CH₂CH₂NH₂(CH₃)₂, -NHSO₂CH₂CH₂NH₂(CH₃)(CH₂CH₃), - NHSO₂CH₂CH₂NH₂(CH₂CH₃)(CH₂CH₃), -NHCH₂CH₂NH₂, -NHCH₂NH₂, - NHCH₂CH₂CH₂NH₂, -NHCH₂CH₂CH₂CH₂NH₂, -NHCH₂CH₂NHCH₃, - NHCH₂CH₂N(CH₃)₂, -N(CH₂CH₂NH₂)₂, -N(CH₂CH₂CH₂NH₂)₂, -N(CH₂NH₂)₂, - N(CH₂NHCH₃)₂, -N(CH₂CH₂NHCH₃)₂, -N(CH₂CH₂CH₂NHCH₃)₂, - NHCONHCH₂CH₂SO₂NH₂, -NHCONHCH₂CH₂CH₂SO₂NH₂, - NHCON(CH₃)(CH₂CH₂CH₂SO₂NH₂), -NHCON(CH₂CH₃)(CH₂CH₂CH₂SO₂NH₂), - NHCON(CH₃)(CH₂CH₂SO₂NH₂), -NHCONHCH₂CH₂SO₂NHCH₃, - NHCONHCH₂CH₂SO₂NHCH₂CH₃, - NHCONHCH₂CH₂SO₂N(CH₃)₂, - NHCH₂CH₂SO₂NH₂, -NHCH₂CH₂CH₂SO₂NH₂, - NHCH₂CH₂CH₂CH₂SO₂NH₂, - NHCH₂CH₂SO₂NHCH₃, -NHCH₂CH₂SO₂N(CH₃)₂.

3. Bisnaphthalimidopropyl (BNIP) derivative of formula I, II, III, IV, V, VI, VII or VIII according to Claim 1, wherein R₁₃, R₁₄, R₁₅, R₁₆, R₁₇, R₁₈, R₁₉, R₂₀, R₂₁ in the molecules of formula II are independently selected from the group of -H, -Cl, -F, -Br, -I, -NH₂, -CH₃, -C(CH₃)₂.

4. Bisnaphthalimidopropyl (BNIP) derivative of formula I, II, III, IV, V, VI, VII or VIII according to Claim 1, wherein R₃₀, R₃₁, R₃₂, R₃₃, R₃₄, R₃₅, R₃₆ in the molecules of formula V are independently selected from the group of -H, -NH₂, -OCH₃, -NO, -Cl, F, I, Br.

5. Bisnaphthalimidopropyl (BNIP) derivative of formula I, II, III, IV, V, VI, VII or VIII according to Claim 1, wherein R₁, R₃₇, R₃₈, R₃₉, R₄₀, R₄₁, R₄₂, R₄₃, R₄₄, R₄₅ in the molecules of formula VI are independently selected from the group of -H, -NH₂, -CH₃, -CH₂CH₃, - CH₂CH₂CH₃, -C(CH₃)₃, Cl, F, Br, I, -OH, -OCH₃, -OCH₂CH₃, -NHCH₃, -NHCH₂CH₃, - N(CH₃)₂, -NO₂, -COOH, -COOCH₃, -COONH₂, -COONHCH₃, -COON(CH₃)₂.

6. Bisnaphthalimidopropyl (BNIP) derivative of formula I, II, III, IV, V, VI, VII or VIII according to Claim 1, wherein R₁, R₄₆, R₄₇, R₄₈, R₄₉, R₅₀, R₅₁, R₅₂, R₅₃, R₅₄ in the molecules of formula VII are independently selected from the group of -H, -NH₂, -CH₃, -CH₂CH₃, - CH₂CH₂CH₃, -C(CH₃)₃, Cl, F, Br, I, -OH, -OCH₃, -OCH₂CH₃, -NHCH₃, -NHCH₂CH₃, - N(CH₃)₂, -NO₂, -COOH, -COOCH₃, -COONH₂, -COONHCH₃, -COON(CH₃)₂.

7. Bisnaphthalimidopropyl (BNIP) derivative of formula I, II, III, IV, V, VI, VII or VIII according to Claim 1, wherein R₁, R₅₅, R₅₆, R₅₇, R₅₈, R₅₉, R₆₀, R₆₁, R₆₂, R₆₃ in the molecules of formula VIII are independently selected from the group of -H, -NH₂, -CH₃, -CH₂CH₃, - CH₂CH₂CH₃, -C(CH₃)₃, Cl, F, Br, I, -OH, -OCH₃, -OCH₂CH₃, -NHCH₃, -NHCH₂CH₃, - N(CH₃)₂, -NO₂, -COOH, -COOCH₃, -COONH₂, -COONHCH₃, -COON(CH₃)₂.

8. Bisnaphthalimidopropyl (BNIP) derivative of formula I, II, III, IV, V, VI, VII or VIII according to Claims 5-7, wherein in molecules of Formula VI, VII, and VIII, R₁ is -H.

9. Method for the preparation of a bisnaphthalimidopropyl (BNIP) derivative of formula I, II, III, IV, V, VI, VII or VIII according to any one of claims 1 -8;
a) Reacting the agent shown with Formula X with paramethyl sulphonyl chloride in the presence of a solvent, 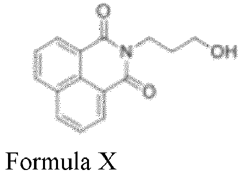
b) Mixing the obtained intermediary product (Formula Y) with NaI in the presence of a solvent and adding a diamino compound and K₂CO₃ solution in a solvent, preferably in acetonitrile 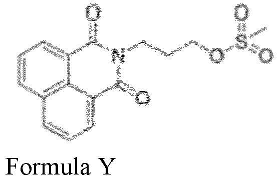
c) isolating the end product from the reaction mixture.

10. A bisnaphthalimidopropyl (BNIP) derivative of formula I, II, III, IV, V, VI, VII or VIII according to any one of Claim 1-8 for use as a drug for treatment of bacterial, viral, parasitic and neoplastic diseases.

11. Bisnaphthalimidopropyl (BNIP) derivative of formula I, II, III, IV, V, VI, VII or VIII for use according to Claim 10, wherein the parasitic disease isschistosomiasis, opisthorchiasis, clonorchiasis, dicrocoeliasis, fascioliasis, paragonimiasis, fasciolopsiasis, echinococcus, teniasis, cysticercosis, difilobotriasis, sparganosis, hymenolepiasis, dracunculiasis, onchocerciasis, filariasis, elephantiasis, mansonellosis, trichinellosis, ankilostomiasis, necatoriasis, ankilostomiasis, ascaridosis, strongyloidiasis, trichuriosis, enterobiasis, anisakiasis, helmintiasis, trichostrongylus, angiostrongylus gnathostomiasis, angiostrongylus, syngamiasis, hirudiniasis, acanthocephaliasis, gongylonemiasis, metastrongylosis, thelaziasis, pediculosis, pityriasis, scabies, myiasis, tungiasis, trombiculosis, scrabiasis, hirudiniasis, linguatulosis, porocephaliasis, leishmaniasis, trypanosomiasis, malaria caused by plasmodium falciparum, malaria caused by plasmodium vivax, malaria caused by plasmodium malariae, chagas disease, toxoplasma, pneumocystosis, babesiosis, piroplasmosis, acanthamoebiasis, naegleriasis and/or microsporidiosis.

12. Bisnaphthalimidopropyl (BNIP) derivative of formula I, II, III, IV, V, VI, VII or VIII for use according to Claim 10 wherein the bacterial disease is bubonic fever, pneumonic fever, cholera, syphilis, congenital syphilis, post streptococcic glomerulonephritis, puerperal fever, impetigo, erysipelas, rheumatic fever, scarlet fever, streptococcal pharyngitis, sepsis, acute bacterial pneumonia, meningitis, otitis media, endometritis, neonatal sepsis, neonatal meningitis, neonatal pneumonia, cystitis, osteomyelitis, toxic shock syndrome, staphylococcal food poisoning, shigellosis, salmonella, paratyphoid, typhoid, rocky mountain spotted fever, urinary tract infections, cornea infections, pneumonia, endocarditis, meningitis, gonorrhea, pelvic inflammatory disease, tuberculosis, leprosy, listeriosis, leptospirosis, legionary disease, peptic ulcer, upper respiratory tract infections, chronic gastritis, bronchitis, septic arthritis, tularemia, bloody diarrhea, urinary tract infections, endocarditis, biliary tract infections, diphtheria, tetanus, parrot disease, trachoma, neonatal conjunctivitis, urethritis, epididymitis, prostatitis, lymphogranulomatosis, atypical pneumonia, Guillain-Barre Syndrome, brucellosis, Lyme disease.

13. Bisnaphthalimidopropyl (BNIP) derivative of formula I, II, III, IV, V, VI, VII or VIII for use according to 10 wherein the viral disease is gastroenteritis, keratoconjunctivitis, pharyngitis, croup, pneumonia, cystitis, foot and mouth disease, pleurodynia, septic meningitis, pericarditis, myocarditis, Burkitt's Lymphoma, Hodgkin's lymphoma, nasopharyngeal carcinoma, acute hepatitis, chronic hepatitis, hepatic cirrhosis, hepatocellular carcinoma, tonsillitis, cytomegalic inclusion disease, Kaposi's sarcoma, Castleman disease, influenza, measles, Reye's syndrome, mumps, anogenital warts, poliomyelitis, rabies, congenital rubella, rubella, smallpox, zoster, congenital smallpox.

14. Bisnaphthalimidopropyl (BNIP) derivative of formula I, II, III, IV, V, VI, VII or VIII for use according to Claim 10, wherein the neoplastic disease is cancer, such as carcinoma, lymphoma, blastoma, sarcoma, leukemia, breast cancer, prostate cancer, colorectal cancer, skin cancer, small cell lung cancer, non-small cell lung cancer, mesothelioma, gastrointestinal cancer, pancreatic cancer, glioblastoma, vulva cancer, cervical cancer, endometrial carcinoma, ovarian cancer, liver cancer, hepatoma , bladder cancer, kidney cancer, salivary gland carcinoma, thyroid cancer and various head and neck cancers .

15. A pharmaceutical composition comprising a bisnaphthalimidopropyl (BNIP) derivative of formula I, II, III, IV, V, VI, VII or VIII according to Claim 1-8.

16. A pharmaceutical composition according to Claim 15, comprising at least one other active agent selected from known active agents that have antiparasitic, antibacterial, antiviral, antineoplastic and/or cytotoxic and/or antimetastatic effects or from combinations comprising two or three of said agents in addition to the bisnaphthalimidopropyl (BNIP) derivative of formula I, II, III, IV, V, VI, VII or VIII

## Patentansprüche

1. Bisnaphthalimidopropyl (BNIP) Derivat von folgenden Formeln I, II, III, IV, V, VI, VII oder VIII;
a) Formel I; 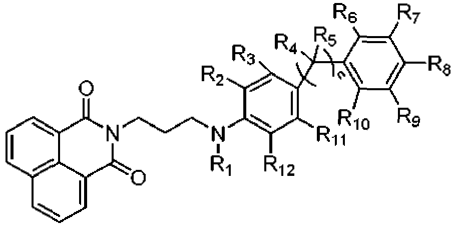
b) Formel II; 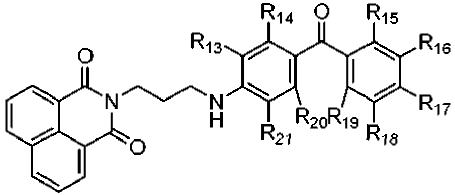
c) Formel III; 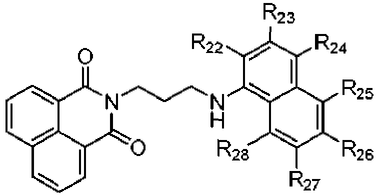
d) Formel IV; 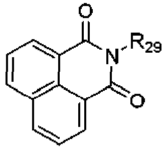
e) Formel V; 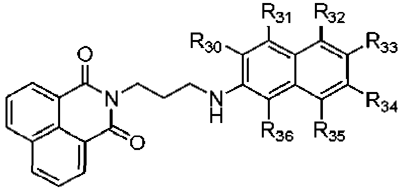
f) Formel VI; 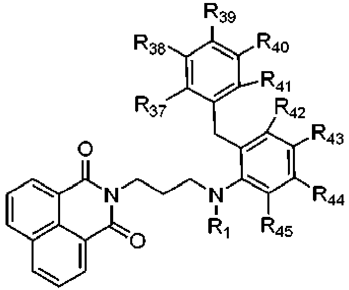
g) Formel VII; 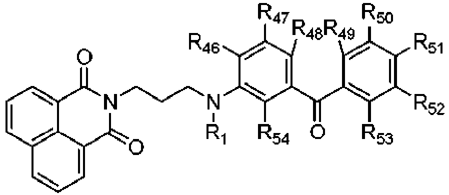
h) Formel VIII; 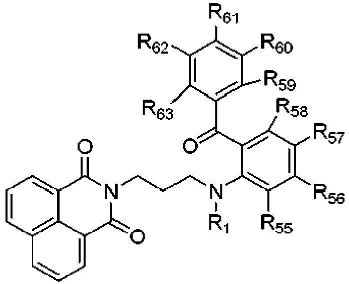
wobei;
-n eine natürliche Zahl zwischen 0 bis 12 ist,
- R₁, R₂, R₃, R₄, R₅, R₆, R₇, R₈, R₉, R₁₀, R₁₁, R₁₂, R₃₇, R₃₈, R₃₉, R₄₀, R₄₁, R₄₂, R₄₃, R₄₄, R₄₅, R₄₆, R₄₇, R₄₈, R₄₉, R₆, R₅₀, R₅₁, R₅₂, R₅₃, R₅₄, R₅₅, R₅₆, R₅₇, R₅₈, R₅₉, R₆₀, R₆₁, R₆₂, R₆₃ unabhängig aus der Gruppe ausgewählt sind, die aus den Folgenden besteht;
-H, -NH₂, -NHR_{X}, -NR_{X}R_{Y}, -NHNR_{X}, -N(R_{X})(NHR_{Y}), -N(R_{X})(NR_{Y}R_{Z}), R_{X}, R_{Y}, R_{Z}, Cl, F, Br, I, -OH, -NO, -NO₂, -COOH, -OR_{X}, -SO₂R_{X}, -SO₂NHR_{X}, -SO₂NR_{X}R_{Y}, -CN, -COOR_{X}, - CONHR_{X}, -CONR_{X}R_{Y}, -NHCOCH₂R_{X}, -NHCOCHR_{X}R_{Y}, -NHCNHR_{X}, -NHCOR_{X}, - NHCONHR_{X}, -NHSO₂R_{X};
- R₁₃, R₁₄, R₁₅, R₁₆, R₁₇, R₁₈, R₁₉, R₂₀, R₂₁ unabhängig aus der Gruppe ausgewählt sind, die aus den Folgenden besteht;
-H, -Cl, -F, -Br, -I, R_{X}, -NH₂, -NHR_{X}, -NR_{X}R_{Y}, -OH, -NO, -NO₂, -COOH, -OR_{X}, - COOR_{X}, -CN;
- R₂₂, R₂₃, R₂₄, R₂₅, R₂₆, R₂₇, R₂₈ unabhängig aus der Gruppe ausgewählt sind, die aus den Folgenden besteht;
-H, -Cl, -F, -Br, -I, R_{X}, -NH₂, -NHR_{X}, -NR_{X}R_{Y}, -OH, -COOH, -OR_{X}, -COOR_{X}, -CN, - SO₃H, -SO₃ Na⁺, 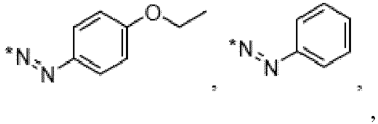 -NO;
-R₂₉ aus der Gruppe ausgewählt ist, die aus den Folgenden besteht; 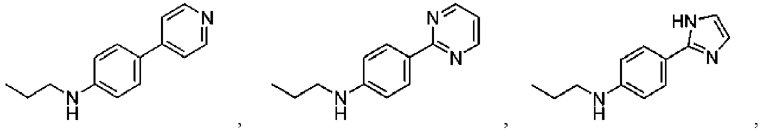 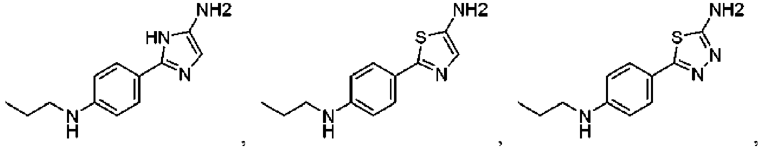 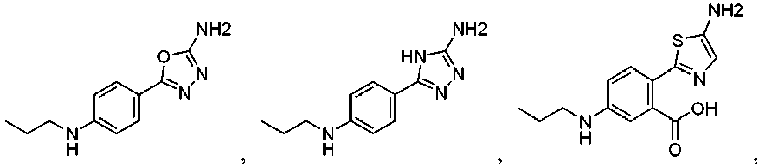 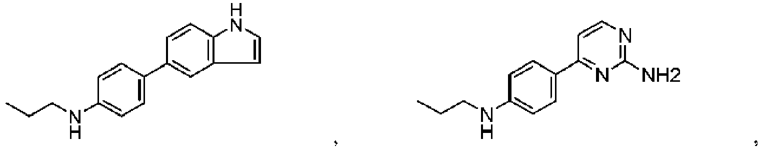 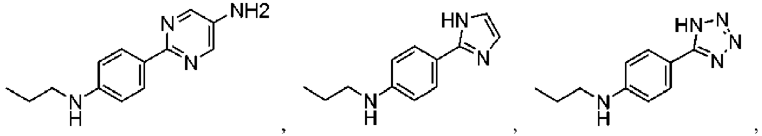 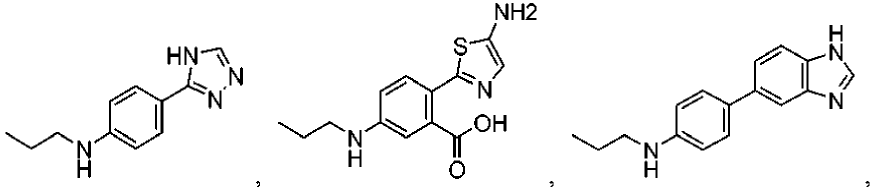 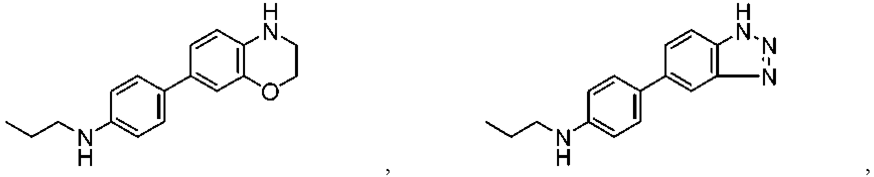 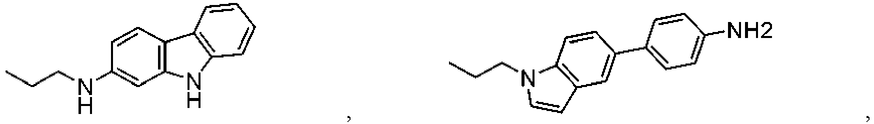 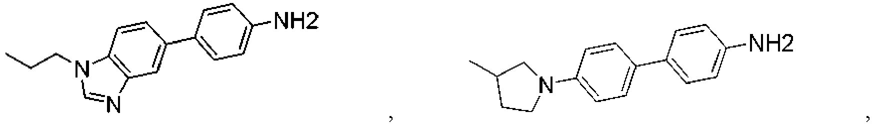 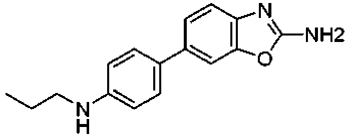
-R₃₀, R₃₁, R₃₂, R₃₃, R₃₄, R₃₅, R₃₆ unabhängig aus der Gruppe ausgewählt sind, die aus den Folgenden besteht;
-H, -Cl, -F, -Br, -I, R_{X}, -NH₂, -NHR_{X}, -NR_{X}R_{Y}, -OH, -NO, -COOH, -OR_{X}, -COOR_{X}, -CN; R_{X}, R_{Y} und R_{Z} unabhängig voneinander aus substituiertem oder unsubstituiertem C1-C12-Alkan, substituiertem oder unsubstituiertem C1-C12-Alken, substituiertem oder unsubstituiertem C1-C12-Alkin, substituiertem oder unsubstituiertem C1-C12-primärem Amin, substituiertem oder unsubstituiertem C1-C12-sekundärem Amin, substituiertem oder unsubstituiertem Alkohol, substituiertem oder unsubstituiertem Alkylcyanid, substituiertem oder unsubstituiertem Sulfonamid, substituiertem oder unsubstituiertem C1-C12-Alkylsulfonyl, substituiertem oder unsubstituiertem C3-C8-Cycloalkyl, substituiertem oder unsubstituiertem Aryl, substituiertem oder unsubstituiertem Heteroaryl und optional und unabhängig voneinander aus einer Gruppe bestehend aus substituierten oder unsubstituierten heterocyclischen oder heteroaromatischen Ringen umfassend ein oder mehr Heteroatom, ausgewählt aus O, S und N, ausgewählt sind.

2. Bisnaphthalimidopropyl (BNIP) Derivat von Formel I, II, III, IV, V, VI, VII oder VIII nach Anspruch 1,
wobei n den Wert 0 oder 1 oder 2 oder 3 oder 4 oder 5 oder 6 oder 7 oder 8 oder 9 oder 10 oder 11 oder 12 in Molekülen von Formel I nimmt;
R₁, R₂, R₃, R₄, R₅, R₆, R₇, R₈, R₉, R₁₀, R₁₁ und R₁₂ unabhängig aus der Gruppe ausgewählt sind, die aus den Folgenden besteht;
-H, -NH₂, -CH₃, -CH₂CH₃, -CH₂CH₂CH₃, -CH₂CH₂CH₂CH₃, -CH₂CH₂CH₂CH₂CH₃, - CH₂CH₂CH₂CH₂CH₂CH₃, CH₂CH₂CH₂CH₂CH₂CH₂CH₃, - CH₂CH₂CH₂CH₂CH₂CH₂CH₂CH₃, CH₂CH₂CH₂CH₂CH₂CH₂CH₂CH₂CH₃, CH₂CH₂CH₂CH₂CH₂CH₂CH₂CH₂CH₂CH₃, - CH₂CH₂CH₂CH₂CH₂CH₂CH₂CH₂CH₂CH₂CH₃, - CH₂CH₂CH₂CH₂CH₂CH₂CH₂CH₂CH₂CH₂CH₂CH₃, -C(CH₃)₃, Cl, F, Br, I, -OH, - OCH₃, -OCH₂CH₃, -OCH₂CH₂CH₃, -OCH₂CH₂CH₂CH₃, -OCH₂CH₂CH₂CH₂CH₃, - OCH₂CH₂CH₂CH₂CH₂CH₃, -NHCH₃, -NHCH₂CH₃, -NHCH₂CH₂CH₃, - NHCH₂CH₂CH₂CH₃, -NHCH₂CH₂CH₂CH₂CH₃, -NHCH₂CH₂CH₂CH₂CH₂CH₃, - N(CH₃)₂, -N(CH₂CH₃)(CH₃), -N(CH₂CH₃)₂, -N(CH₂CH₃)(CH₂CH₂CH₃), - N(CH₃)(CH₂CH₂CH₃), N(CH₂CH₂CH₃)₂, -NHNH₂, -NHNHCH₃, -NHNHCH₂CH₃, - NHNHCH₂CH₂CH₃, -NHN(CH₃)₂, -NHN(CH₂CH₃)₂, -NHN(CH₃)(CH₂CH₃), - N(CH₃)(NHCH₃), -N(CH₃)(N(CH₃)₂), -COOH, -SO₂CH₃, -SO₂CH₂CH₂, - SO₂CH₂CH₂CH₃, -SO₂CH₂CH₂CH₂CH₃, -SO₂CH₂CH₂CH₂CH₂CH₃, - SO₂CH₂CH₂CH₂CH₂CH₂CH₃, -CN, -CONH₂, -CONHCH₃, -CONHCH₂CH₃, - CONHCH₂CH₂CH₃, -CONHCH₂CH₂CH₂CH₃, -CONHCH₂CH₂CH₂CH₂CH₃, - CONHCH₂CH₂CH₂CH₂CH₂CH₃, -CON(CH₃)₂, -CON(CH₃)(CH₂CH₃), - CON(CH₂CH₃)₂, -CON(CH₂CH₃)(CH₂CH₂CH₃), -CON(CH₃)(CH₂CH₂CH₃), - CON(CH₂CH₂CH₃)₂, -COCH₃, -COCH₂CH₃, -COCH₂CH₂CH₃, - COCH₂CH₂CH₂CH₃, -COCH₂CH₂CH₂CH₂CH₃, -COCH₂CH₂CH₂CH₂CH₂CH₃, - COOCH₃, -COOCH₂CH₃, -COOCH₂CH₂CH₃, -COOCH₂CH₂CH₂CH₃, - COOCH₂CH₂CH₂CH₂CH₃, -COOCH₂CH₂CH₂CH₂CH₂CH₃, -COCH(CH₃)₂, - COC(CH₃)₃, -COCH(CH₂CH₃)₂, -COCH(CH₃)(CH₂CH₃), -COCH₂CH₂CH₃, - COCH₂CH₂CH₂CH₃, -COCH₂CH₂CH₂CH₂CH₃, -COCH₂CH₂CH₂CH₂CH₂CH₃, - SO₂NH₂, SO₂NHCH₃, SO₂NHCH₂CH₃, -SO₂NHCH₂CH₂CH₃, SO₂N(CH₃)₂, - SO₂N(CH₃)(CH₂CH₃), -SO₂N(CH₂CH₃)₂, -SO₂N(CH₃)(CH₂CH₂CH₃), - SO₂N(CH₂CH₂CH₃)₂, -NHCONHCH₃, -NHCON(CH₃)₂, -NHCONHCH₂CH₃, NHCON(CH₃)(CH₂CH₃), -NHCON(CH₂CH₃)₂, -NHCOCH(CH₃)(NH₂), - NHCOCH(CH₃)₂, -NHCOCH(CH₃)(NHCH₃), -NHCOCH(CH₃)(N(CH₃)₂),-NHCOCH(CH₂CH₃)₂, -NHCOCH(CH₂OH)(NH₂), -NHCOCH(CH₂CH₂OH)(NH₂), - NHCOCH(CH₂CH₂OH)(NHCH₃), -NHCOCH(CH₂OH)(NHCH₃), NHCOCH(CH₂OH)(N(CH₃)₂), NHCOCH(CH₂OH)(N(CH₂CH₃)₂), - NHCOCH(CH₂SH)(NH₂), -NHCOCH(CH₂CH₂SH)(NH₂), - NHCOCH(CH₂CH₂CH₂SH)(NH₂), -NHCOCH(CH₂SH)(NHCH₃), - NHCOCH(CH₂SH)(NHCH₂CH₃), -NHCOCH(CH₂SH)(N(CH₃)₂), 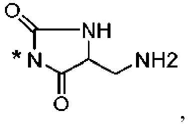 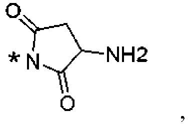 -NH(CNH)NH₂, -NH(CN(CH₃))NH₂, -NH(CN(CH₂CH₃))NH₂, - NH(CN(CH₃))NH(CH₃), -- NH(CN(CH₃))N(CH₃)₂, -NH(CNH)NHCH₃, NH(CNH)N(CH₃)₂,-NH(CO)NHCH₂CH₂NH₂,-NH(CO)NHCH₂NH₂, - NH(CO)NHCH₂CH₂NHCH₃,-NH(CO)N(CH₃)(CH₂CH₂NH₂), - NH(CO)CH₂CH₂NH₂,-NH(CO)CH₂NH₂,-NH(CO)CH₂CH₂CH₂NH₂, - NH(CO)CH₂CH₂CH₂CH₂NH₂, -NH(CO)CH₂CH₂NHCH₃, -NH(CO)CH₂CH₂N(CH₃)₂, -NH(CO)CH₂CH₂NH(CH₂CH₃), -NH(CO)CH₂CH₂N(CH₃)(CH₂CH₃), - NHSO₂CH₂CH₂NH₂, -NHSO₂CH₂CH₂CH₂NH₂, -NHSO₂CH₂CH₂NH₂HCH₃, - NHSO₂CH₂CH₂NH₂(CH₃)₂, -NHSO₂CH₂CH₂NH₂(CH₃)(CH₂CH₃), - NHSO₂CH₂CH₂NH₂(CH₂CH₃)(CH₂CH₃), -NHCH₂CH₂NH₂, -NHCH₂NH₂, - NHCH₂CH₂CH₂NH₂, -NHCH₂CH₂CH₂CH₂NH₂, -NHCH₂CH₂NHCH₃, - NHCH₂CH₂N(CH₃)₂, -N(CH₂CH₂NH₂)₂, -N(CH₂CH₂CH₂NH₂)₂, -N(CH₂NH₂)₂, - N(CH₂NHCH₃)₂, -N(CH₂CH₂NHCH₃)₂, -N(CH₂CH₂CH₂NHCH₃)₂, - NHCONHCH₂CH₂SO₂NH₂, -NHCONHCH₂CH₂CH₂SO₂NH₂, - NHCON(CH₃)(CH₂CH₂CH₂SO₂NH₂), -NHCON(CH₂CH₃)(CH₂CH₂CH₂SO₂NH₂), - NHCON(CH₃)(CH₂CH₂SO₂NH₂), -NHCONHCH₂CH₂SO₂NHCH₃, - NHCONHCH₂CH₂SO₂NHCH₂CH₃, - NHCONHCH₂CH₂SO₂N(CH₃)₂, - NHCH₂CH₂SO₂NH₂, -NHCH₂CH₂CH₂SO₂NH₂, - NHCH₂CH₂CH₂CH₂SO₂NH₂, - NHCH₂CH₂SO₂NHCH₃, -NHCH₂CH₂SO₂N(CH₃)₂.

3. Bisnaphthalimidopropyl (BNIP) Derivat von Formel I, II, III, IV, V, VI, VII oder VIII nach Anspruch 1, wobei R₁₃, R₁₄, R₁₅, R₁₆, R₁₇, R₁₈, R₁₉, R₂₀, R₂₁ in Molekülen von Formel II unabhängig voneinander aus der Gruppe bestehend aus -H, -Cl, -F, -Br, -I, -NH₂, -CH₃, - C(CH₃)₂ ausgewählt sind.

4. Bisnaphthalimidopropyl (BNIP) Derivat von Formel I, II, III, IV, V, VI, VII oder VIII nach Anspruch 1, wobei R₃₀, R₃₁, R₃₂, R₃₃, R₃₄, R₃₅, R₃₆ in Molekülen von Formel V unabhängig voneinander aus der Gruppe bestehend aus -H, -NH₂, -OCH₃, -NO, -Cl, F, I, Br ausgewählt sind.

5. Bisnaphthalimidopropyl (BNIP) Derivat von Formel I, II, III, IV, V, VI, VII oder VIII nach Anspruch 1, wobei R₁, R₃₇, R₃₈, R₃₉, R₄₀, R₄₁, R₄₂, R₄₃, R₄₄, R₄₅ in Molekülen von Formel VI unabhängig voneinander aus der Gruppe bestehend aus -H, -NH₂, -CH₃, - CH₂CH₃, -CH₂CH₂CH₃, -C(CH₃)₃, Cl, F, Br, I, -OH, -OCH₃, -OCH₂CH₃, -NHCH₃, - NHCH₂CH₃, -N(CH₃)₂, -NO₂, -COOH, -COOCH₃, -COONH₂, -COONHCH₃, - COON(CH₃)₂ ausgewählt sind.

6. Bisnaphthalimidopropyl (BNIP) Derivat von Formel I, II, III, IV, V, VI, VII oder VIII nach Anspruch 1, wobei R₁, R₄₆, R₄₇, R₄₈, R₄₉, R₅₀, R₅₁, R₅₂, R₅₃, R₅₄ in Molekülen von Formel VII unabhängig voneinander aus der Gruppe bestehend aus -H, -NH₂, -CH₃, - CH₂CH₃, -CH₂CH₂CH₃, -C(CH₃)₃, Cl, F, Br, I, -OH, -OCH₃, -OCH₂CH₃, -NHCH₃, - NHCH₂CH₃, -N(CH₃)₂, -NO₂, -COOH, -COOCH₃, -COONH₂, -COONHCH₃, - COON(CH₃)₂ ausgewählt sind.

7. Bisnaphthalimidopropyl (BNIP) Derivat von Formel I, II, III, IV, V, VI, VII oder VIII nach Anspruch 1, wobei R₁, R₅₅, R₅₆, R₅₇, R₅₈, R₅₉, R₆₀, R₆₁, R₆₂, R₆₃ in Molekülen von Formel VIII unabhängig voneinander aus der Gruppe bestehend aus -H, -NH₂, -CH₃, - CH₂CH₃, -CH₂CH₂CH₃, -C(CH₃)₃, Cl, F, Br, I, -OH, -OCH₃, -OCH₂CH₃, -NHCH₃, - NHCH₂CH₃, -N(CH₃)₂, -NO₂, -COOH, -COOCH₃, -COONH₂, -COONHCH₃, - COON(CH₃)₂ ausgewählt sind.

8. Bisnaphthalimidopropyl (BNIP) Derivat von Formel I, II, III, IV, V, VI, VII oder VIII nach Ansprüche 5 bis 7, wobei R₁ in Molekülen von Formel VI, VII und VIII -H ist.

9. Verfahren zur Vorbereitung von Bisnaphthalimidopropyl (BNIP) Derivat von Formel I, II, III, IV, V, VI, VII oder VIII nach einem der Ansprüche 1 bis 8;
a) Reagieren der Substanz dargestellt in der Formel X mit Paramethyl Sulfonylchlorid in Gegenwart eines Lösungsmittels, 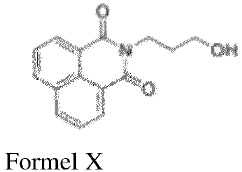
b) Mischen des erhaltenen Zwischenprodukts (Formel Y) mit NaI in Gegenwart eines Lösungsmittels und Hinzufügen einer Diaminoverbindung und K₂CO₃ Lösungsmittels in einem Lösungsmittel, vorzugsweise in Acetonitril 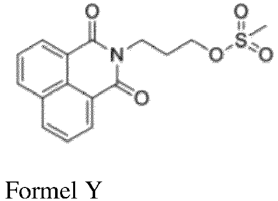
c) Isolieren des Endprodukts von Reaktionsgemisch.

10. Bisnaphthalimidopropyl (BNIP) Derivat von Formel I, II, III, IV, V, VI, VII oder VIII nach einem der Ansprüche 1 bis 8 zur Verwendung als ein Medikament für Behandlung der bakteriellen, viralen, parasitären und neoplastischen Erkrankungen.

11. Bisnaphthalimidopropyl (BNIP) Derivat von Formel I, II, III, IV, V, VI, VII oder VIII nach Anspruch 10, wobei die parasitäre Erkrankung Schistosomiasis, Opisthorchiasis, Clonorchiasis, Dicrocoeliasis, Faszioliasis, Paragonimiasis, Fasciolopsiasis, Echinokokke, Taeniasis, Zystizerkose, Diphyllobothriasis, Sparganose, Hymenolepiasis, Drakunkulose, Onchozerkose, Filariose, Elephantiasis, Mansonellose, Trichinellose, Ankylostomiasis, Nekatoriasis, Ankylostomiasis, Askaridose, Strongyloidiasis, Trichuriasis, Enterobiasis, Anisakiasis, Helminthiasis, Trichostrongylus, Angiostrongylus Gnathostomiasis, Angiostrongylus, Syngamiasis, Hirudiniasis, Akanthozephaliasis, Gongylonemiasis, Metastrongylose, Thelazia, Pedikulose, Pityriasis, Skabies, Myiasis, Tungiasis, Trombiculosis, Scrabiasis, Hirudiniasis, Linguatulosis, Porocephaliasis, Leishmaniose, Trypanosomiasis, Malaria verursacht durch Plasmodium falciparum, Malaria verursacht durch Plasmodium vivax, Malaria verursacht durch Plasmodium malariae, Chagas-Krankheit, Toxoplasma, Pneumozystose, Babesiose, Piroplasmose, Acanthamoeba, Naegleriasis und/oder Mikrosporidiose ist.

12. Bisnaphthalimidopropyl (BNIP) Derivat von Formel I, II, III, IV, V, VI, VII oder VIII nach Anspruch 10, wobei die Bakterienerkrankung Beulenpest, Lungenpest, Cholera, Syphilis, angeborene Syphilis, Poststreptokokken-Glomerulonephritis, Kindbettfieber, Impetigo, Erysipel, rheumatisches Fieber, Scharlachfieber, Streptokokkenpharyngitis, Sepsis, akute bakterielle Pneumonie, Meningitis, Mittelohrentzündung, Endometritis, neonatale Sepsis, neonatale Meningitis, Neugeborenenpneumonie, Zystitis, Osteomyelitis, toxisches Schocksyndrom, Staphylokokken-Lebensmittelvergiftung, Shigellose, Salmonellen, Paratyphus, Typhus, Felsengebirgsfleckfieber, Harnwegsinfektionen, Hornhautinfektionen, Lungenentzündung, Endokarditis, Meningitis, Gonorrhoe, entzündliche Beckenerkrankung, Tuberkulose, Lepra, Listeriose, Leptospirose, Legionärskrankheit, Magengeschwür, Infektionen der oberen Atemwege, chronischer Gastritis, Bronchitis, septische Arthritis, Tularämie, blutiger Durchfall, Harnwegsinfektionen, Endokarditis, Gallenwegeinfektionen, Diphtherie, Tetanus, Papageienkrankheit, Trachom, Neugeborenenkonjunktivitis, Urethritis, Epididymitis, Prostatitis, Lymphogranulomatose, atypische Pneumonie, Guillain-Barre-Syndrom, Brucellose, Lyme-Borreliose ist.

13. Bisnaphthalimidopropyl (BNIP) Derivat von Formel I, II, III, IV, V, VI, VII oder VIII nach Anspruch 10, wobei die Viruserkrankung Gastroenteritis, Keratoconjunctivitis, Pharyngitis, Krupp, Lungenentzündung, Zystitis, Maul-und-Klauenseuche, Pleurodynie, bakterielle Meningitis, Perikarditis, Myokarditis, Burkitt-Lymphom, Hodgkin-Lymphom, Nasopharynxkarzinom, akute Hepatitis, chronische Hepatitis, Leberzirrhose, hepatozelluläres Karzinom, Tonsillitis, Zytomegalie-Syndrom, Kaposi-Sarkom, Castleman-Krankheit, Influenza, Masern, Reye-Syndrom, Mumps, anogenitale Warzen, Poliomyelitis, Tollwut, Rötelnembryofetopathie, Röteln, Pocken, Zoster, kongenitale Pocken ist.

14. Bisnaphthalimidopropyl (BNIP) Derivat von Formel I, II, III, IV, V, VI, VII oder VIII nach Anspruch 10, wobei die neoplastische Erkrankung der Krebs, beispielsweise Karzinom, Lymphom, Blastom, Sarkom, Leukämie, Brustkrebs, Prostatakrebs, Darmkrebs, Hautkrebs, kleinzelliger Lungenkrebs, nicht-kleinzelliger Lungenkrebs, Mesotheliom, gastrointestinaler Krebs, Bauchspeicheldrüsenkrebs, Glioblastom, Vulvakrebs, Gebärmutterhalskrebs, Endometriumkarzinom, Ovarialkarzinom, Leberkrebs, Hepatom, Blasenkrebs, Nierenkrebs, Speicheldrüsenkarzinom, Schilddrüsenkrebs und diverse Kopf- und Halskrebse ist.

15. Pharmazeutische Zusammensetzung umfassend ein Bisnaphthalimidopropyl (BNIP) Derivat von Formel I, II, III, IV, V, VI, VII oder VIII nach Ansprüche 1 bis 8.

16. Pharmazeutische Zusammensetzung nach Anspruch 15 umfassend mindestens einen anderen Wirkstoff, ausgewählt aus aktiven Wirkstoffen, die dafür bekannt sind, dass sie antiparasitäre, antibakterielle, antivirale, antineoplastische und/oder cytotoxische und/oder antimetastatische Wirkungen aufweisen, oder aus den Kombinationen umfassend zwei oder drei der genannten Wirkstoffen zusätzlich zum Bisnaphthalimidopropyl (BNIP) Derivat von Formel I, II, III, IV, V, VI, VII oder VIII.

## Revendications

1. Dérivé de bisnaphtalimidopropyl (BNIP) de Formule I, II, III, IV, V, VI, VII ou VIII ;
a) Formule I; 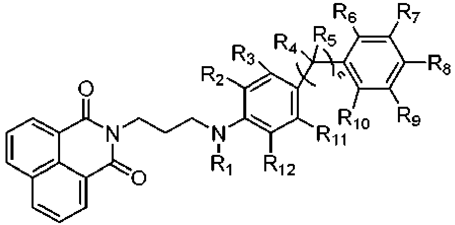
b) Formule II; 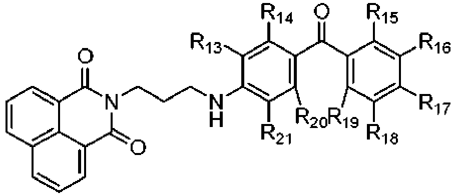
c) Formule III; 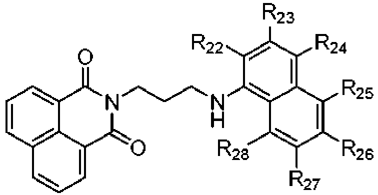
d) Formule IV; 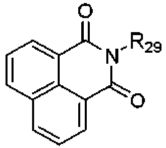
e) Formule V; 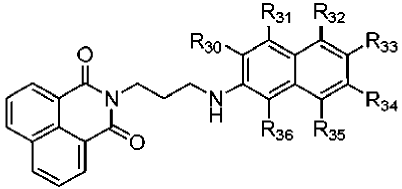
f) Formule VI; 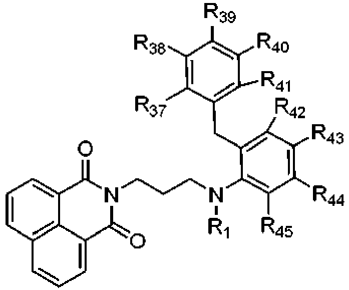
g) Formule VII; 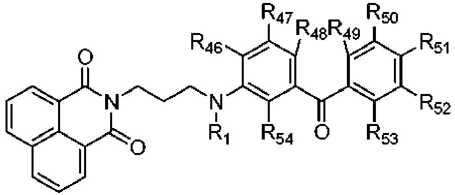
h) Formule VIII; 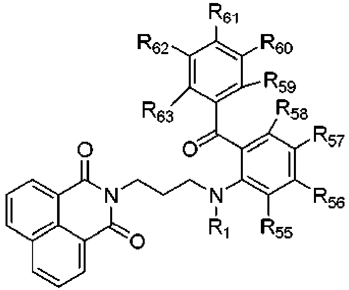
dans lequel;
- n est un nombre naturel compris entre 0 et 12,
- R₁, R₂, R₃, R₄, R₅, R₆, R₇, R₈, R₉, R₁₀, R₁₁, R₁₂, R₃₇, R₃₈, R₃₉, R₄₀, R₄₁, R₄₂, R₄₃, R₄₄, R₄₅, R₄₆, R₄₇, R₄₈, R₄₉, R₅₀, R₅₁, R₅₂, R₅₃, R₅₄, R₅₅, R₅₆, R₅₇, R₅₈, R₅₉, R₆₀, R₆₁, R₆₂, R₆₃, sont indépendamment choisis dans le groupe constitué par ;
-H, -NH₂, -NHR_{X}, -NR_{X}R_{Y}, -NHNR_{X}, -N(R_{X})(NHR_{Y}), -N(R_{X})(NR_{Y}R_{Z}), R_{X}, R_{Y}, R_{Z}, Cl, F, Br, I, -OH, -NO, -NO₂, -COOH, -OR_{X}, -SO₂R_{X}, -SO₂NHR_{X}, -SO₂NR_{X}R_{Y}, -CN, -COOR_{X}, - CONHR_{X}, -CONR_{X}R_{Y}, -NHCOCH₂R_{X}, -NHCOCHR_{X}R_{Y}, -NHCNHR_{X}, -NHCOR_{X}, - NHCONHR_{X}, -NHSO₂R_{X};
- R₁₃, R₁₄, R₁₅, R₁₆, R₁₇, R₁₈, R₁₉, R₂₀, R₂₁ sont indépendamment choisis dans le groupe constitué par ;
-H, -Cl, -F, -Br, -I, R_{X}, -NH₂, -NHR_{X}, -NR_{X}R_{Y}, -OH, -NO, -NO₂, -COOH, -OR_{X}, -COOR_{X}, - CN;
- R₂₂, R₂₃, R₂₄, R₂₅, R₂₆, R₂₇, R₂₈ sont indépendamment choisis dans le groupe constitué par -H, -Cl, -F, -Br, -I, R_{X}, -NH₂, -NHR_{X}, -NR_{X}R_{Y}, -OH, -COOH, -OR_{X}, -COOR_{X}, -CN, - SO₃H, -SO₃ Na⁺, 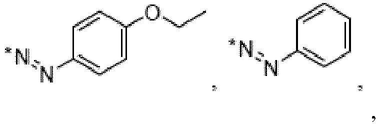 -NO;
-R₂₉ est choisi dans le groupe constitué par; 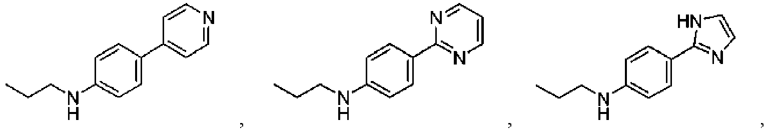 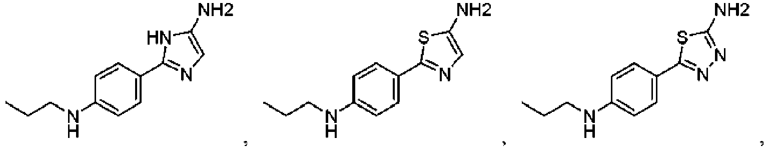 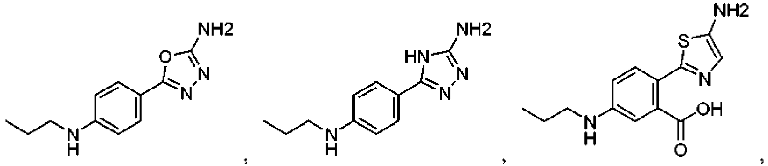 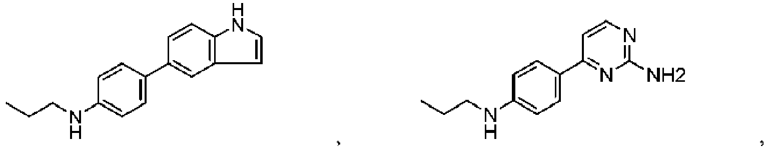 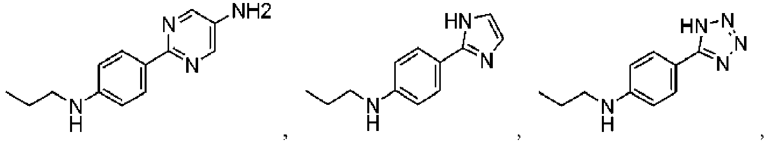 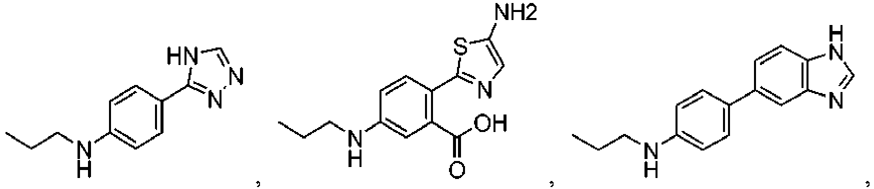 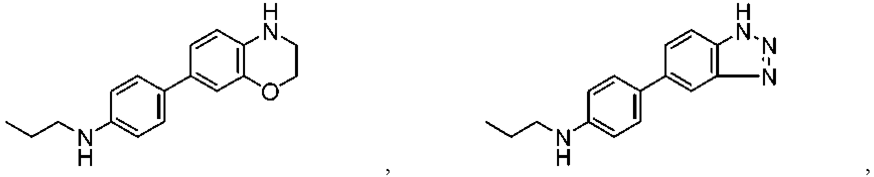 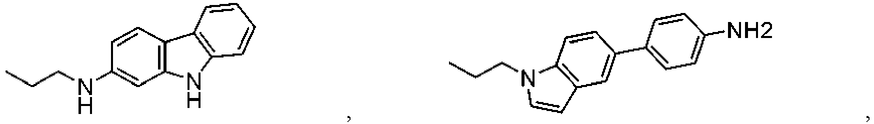 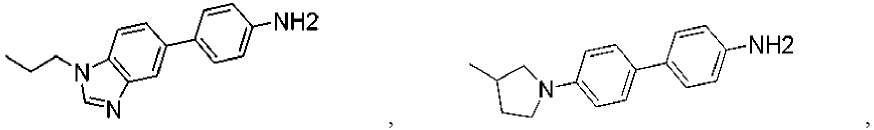 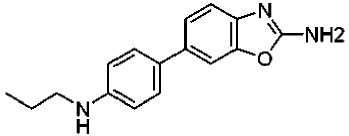
- R₃₀, R₃₁, R₃₂, R₃₃, R₃₄, R₃₅, R₃₆ sont indépendamment choisis dans le groupe constitué par ;
-H, -Cl, -F, -Br, -I, R_{X}, -NH₂, -NHR_{X}, -NR_{X}R_{Y}, -OH, -NO, -COOH, -OR_{X}, -COOR_{X}, -CN;
R_{X}, R_{Y}, et R_{Z} sont choisis indépendamment parmi alcane en C1-C12 substitué ou non substitué, alcène en C1-C12 substitué ou non substitué, alcyne en C1-C12 substitué ou non substitué, amine primaire en C1-C12 substituée ou non, amine secondaire en C1-C12 substituée ou non, alcool substitué ou non substitué, cyanure d'alkyle substitué ou non substitué, sulfonamide substitué ou non substitué, alkyle sulfonyle en C1-C12 substitué ou non substitué, cycloalkyle en C3-C8 substitué ou non substitué, aryle substitué ou non substitué, hétéroaryle substitué ou non substitué, optionnellement et indépendamment les uns des autres d'un groupe formé d'anneaux hétérocycliques ou hétéroaromatiques substitués ou non substitués qui comprennent un ou plusieurs hétéroatomes choisis parmi O, S et N.

2. Dérivé de bisnaphtalimidopropyl (BNIP) de Formule I, II, III, IV, V, VI, VII ou VIII selon la revendication 1,
dans lequel n prend la valeur de 0 ou 1 ou 2 ou 3 ou 4 ou 5 ou 6 ou 7 ou 8 ou 9 ou 10 ou 11 ou 12 dans les molécules de la Formule I;
R₁, R₂, R₃, R₄, R₅, R₆, R₇, R₈, R₉, R₁₀, R₁₁ et R₁₂ sont indépendamment choisis dans le groupe constitué par ;
-H, -NH₂, -CH₃, -CH₂CH₃, -CH₂CH₂CH₃, -CH₂CH₂CH₂CH₃, -CH₂CH₂CH₂CH₂CH₃, - CH₂CH₂CH₂CH₂CH₂CH₃, CH₂CH₂CH₂CH₂CH₂CH₂CH₃, - CH₂CH₂CH₂CH₂CH₂CH₂CH₂CH₃, -CH₂CH₂CH₂CH₂CH₂CH₂CH₂CH₂CH₃, CH₂CH₂CH₂CH₂CH₂CH₂CH₂CH₂CH₂CH₃, - CH₂CH₂CH₂CH₂CH₂CH₂CH₂CH₂CH₂CH₂CH₃, - CH₂CH₂CH₂CH₂CH₂CH₂CH₂CH₂CH₂CH₂CH₂CH₃, -C(CH₃)₃, Cl, F, Br, I, -OH, - OCH₃, -OCH₂CH₃, -OCH₂CH₂CH₃, -OCH₂CH₂CH₂CH₃, -OCH₂CH₂CH₂CH₂CH₃, - OCH₂CH₂CH₂CH₂CH₂CH₃, -NHCH₃, -NHCH₂CH₃, -NHCH₂CH₂CH₃, - NHCH₂CH₂CH₂CH₃, -NHCH₂CH₂CH₂CH₂CH₃, -NHCH₂CH₂CH₂CH₂CH₂CH₃, - N(CH₃)₂, -N(CH₂CH₃)(CH₃), -N(CH₂CH₃)₂, -N(CH₂CH₃)(CH₂CH₂CH₃), - N(CH₃)(CH₂CH₂CH₃), N(CH₂CH₂CH₃)₂, -NHNH₂, -NHNHCH₃, -NHNHCH₂CH₃, - NHNHCH₂CH₂CH₃, -NHN(CH₃)₂, -NHN(CH₂CH₃)₂, -NHN(CH₃)(CH₂CH₃), - N(CH₃)(NHCH₃), -N(CH₃)(N(CH₃)₂), -COOH, -SO₂CH₃, -SO₂CH₂CH₂, - SO₂CH₂CH₂CH₃, -SO₂CH₂CH₂CH₂CH₃, -SO₂CH₂CH₂CH₂CH₂CH₃, - SO₂CH₂CH₂CH₂CH₂CH₂CH₃, -CN, -CONH₂, -CONHCH₃, -CONHCH₂CH₃, - CONHCH₂CH₂CH₃, -CONHCH₂CH₂CH₂CH₃, -CONHCH₂CH₂CH₂CH₂CH₃, - CONHCH₂CH₂CH₂CH₂CH₂CH₃, -CON(CH₃)₂, -CON(CH₃)(CH₂CH₃), - CON(CH₂CH₃)₂, -CON(CH₂CH₃)(CH₂CH₂CH₃), -CON(CH₃)(CH₂CH₂CH₃), - CON(CH₂CH₂CH₃)₂, -COCH₃, -COCH₂CH₃, -COCH₂CH₂CH₃, - COCH₂CH₂CH₂CH₃, -COCH₂CH₂CH₂CH₂CH₃, -COCH₂CH₂CH₂CH₂CH₂CH₃, - COOCH₃, -COOCH₂CH₃, -COOCH₂CH₂CH₃, -COOCH₂CH₂CH₂CH₃, - COOCH₂CH₂CH₂CH₂CH₃, -COOCH₂CH₂CH₂CH₂CH₂CH₃, -COCH(CH₃)₂, - COC(CH₃)₃, -COCH(CH₂CH₃)₂, -COCH(CH₃)(CH₂CH₃), -COCH₂CH₂CH₃, - COCH₂CH₂CH₂CH₃, -COCH₂CH₂CH₂CH₂CH₃, -COCH₂CH₂CH₂CH₂CH₂CH₃, - SO₂NH₂, SO₂NHCH₃, SO₂NHCH₂CH₃, -SO₂NHCH₂CH₂CH₃, SO₂N(CH₃)₂, - SO₂N(CH₃)(CH₂CH₃), -SO₂N(CH₂CH₃)₂, -SO₂N(CH₃)(CH₂CH₂CH₃), - SO₂N(CH₂CH₂CH₃)₂, -NHCONHCH₃, -NHCON(CH₃)₂, -NHCONHCH₂CH₃, NHCON(CH₃)(CH₂CH₃), -NHCON(CH₂CH₃)₂, -NHCOCH(CH₃)(NH₂),-NHCOCH(CH₃)₂, -NHCOCH(CH₃)(NHCH₃), -NHCOCH(CH₃)(N(CH₃)₂), - NHCOCH(CH₂CH₃)₂, -NHCOCH(CH₂OH)(NH₂), -NHCOCH(CH₂CH₂OH)(NH₂), - NHCOCH(CH₂CH₂OH)(NHCH₃), -NHCOCH(CH₂OH)(NHCH₃), NHCOCH(CH₂OH)(N(CH₃)₂), NHCOCH(CH₂OH)(N(CH₂CH₃)₂), - NHCOCH(CH₂SH)(NH₂), -NHCOCH(CH₂CH₂SH)(NH₂), - NHCOCH(CH₂CH₂CH₂SH)(NH₂), -NHCOCH(CH₂SH)(NHCH₃), - NHCOCH(CH₂SH)(NHCH₂CH₃), -NHCOCH(CH₂SH)(N(CH₃)₂), 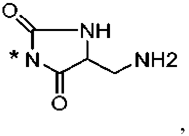 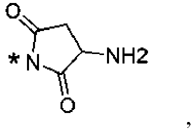 -NH(CNH)NH₂, -NH(CN(CH₃))NH₂, -NH(CN(CH₂CH₃))NH₂, - NH(CN(CH₃))NH(CH₃), -- NH(CN(CH₃))N(CH₃)₂, -NH(CNH)NHCH₃, NH(CNH)N(CH₃)₂, -NH(CO)NHCH₂CH₂NH₂, -NH(CO)NHCH₂NH₂, - NH(CO)NHCH₂CH₂NHCH₃, -NH(CO)N(CH₃)(CH₂CH₂NH₂), - NH(CO)CH₂CH₂NH₂, -NH(CO)CH₂NH₂, -NH(CO)CH₂CH₂CH₂NH₂, - NH(CO)CH₂CH₂CH₂CH₂NH₂, -NH(CO)CH₂CH₂NHCH₃, -NH(CO)CH₂CH₂N(CH₃)₂, -NH(CO)CH₂CH₂NH(CH₂CH₃), -NH(CO)CH₂CH₂N(CH₃)(CH₂CH₃), - NHSO₂CH₂CH₂NH₂, -NHSO₂CH₂CH₂CH₂NH₂, -NHSO₂CH₂CH₂NH₂HCH₃, - NHSO₂CH₂CH₂NH₂(CH₃)₂, -NHSO₂CH₂CH₂NH₂(CH₃)(CH₂CH₃), - NHSO₂CH₂CH₂NH₂(CH₂CH₃)(CH₂CH₃), -NHCH₂CH₂NH₂, -NHCH₂NH₂, - NHCH₂CH₂CH₂NH₂, -NHCH₂CH₂CH₂CH₂NH₂, -NHCH₂CH₂NHCH₃, - NHCH₂CH₂N(CH₃)₂, -N(CH₂CH₂NH₂)₂, -N(CH₂CH₂CH₂NH₂)₂, -N(CH₂NH₂)₂, - N(CH₂NHCH₃)₂, -N(CH₂CH₂NHCH₃)₂, -N(CH₂CH₂CH₂NHCH₃)₂, - NHCONHCH₂CH₂SO₂NH₂, -NHCONHCH₂CH₂CH₂SO₂NH₂, - NHCON(CH₃)(CH₂CH₂CH₂SO₂NH₂), -NHCON(CH₂CH₃)(CH₂CH₂CH₂SO₂NH₂), - NHCON(CH₃)(CH₂CH₂SO₂NH₂), -NHCONHCH₂CH₂SO₂NHCH₃, - NHCONHCH₂CH₂SO₂NHCH₂CH₃, - NHCONHCH₂CH₂SO₂N(CH₃)₂, - NHCH₂CH₂SO₂NH₂, -NHCH₂CH₂CH₂SO₂NH₂, - NHCH₂CH₂CH₂CH₂SO₂NH₂, - NHCH₂CH₂SO₂NHCH₃, -NHCH₂CH₂SO₂N(CH₃)₂.

3. Dérivé de bisnaphtalimidopropyl (BNIP) de Formule I, II, III, IV, V, VI, VII ou VIII selon la revendication 1, dans lequel R₁₃, R₁₄, R₁₅, R₁₆, R₁₇, R₁₈, R₁₉, R₂₀, R₂₁ dans les molécules de la Formule II sont choisis indépendamment dans le groupe constitué par -H, -Cl, -F, -Br, -I, - NH₂, -CH₃, -C(CH₃)₂.

4. Dérivé de bisnaphtalimidopropyl (BNIP) de Formule I, II, III, IV, V, VI, VII ou VIII selon la revendication 1, dans lequel R₃₀, R₃₁, R₃₂, R₃₃, R₃₄, R₃₅, R₃₆ dans les molécules de la Formule V sont choisis indépendamment dans le groupe constitué par -H, -NH₂, -OCH₃, -NO, -Cl, F, I, Br.

5. Dérivé de bisnaphtalimidopropyl (BNIP) de Formule I, II, III, IV, V, VI, VII ou VIII selon la revendication 1, dans lequel R₁, R₃₇, R₃₈, R₃₉, R₄₀, R₄₁, R₄₂, R₄₃, R₄₄, R₄₅ dans les molécules de la Formule VI sont choisis indépendamment dans le groupe constitué par -H, -NH₂, -CH₃, - CH₂CH₃, -CH₂CH₂CH₃, -C(CH₃)₃, Cl, F, Br, I, -OH, -OCH₃, -OCH₂CH₃, -NHCH₃, - NHCH₂CH₃, -N(CH₃)₂, -NO₂, -COOH, -COOCH₃, -COONH₂, -COONHCH₃, -COON(CH₃)₂.

6. Dérivé de bisnaphtalimidopropyl (BNIP) de Formule I, II, III, IV, V, VI, VII ou VIII selon la revendication 1, dans lequel R₁, R₄₆, R₄₇, R₄₈, R₄₉, R₅₀, R₅₁, R₅₂, R₅₃, R₅₄ dans les molécules de la Formule VII sont choisis indépendamment dans le groupe constitué par -H, -NH₂, -CH₃, -CH₂CH₃, -CH₂CH₂CH₃, -C(CH₃)₃, Cl, F, Br, I, -OH, -OCH₃, -OCH₂CH₃, -NHCH₃, - NHCH₂CH₃, -N(CH₃)₂, -NO₂, -COOH, -COOCH₃, -COONH₂, -COONHCH₃, -COON(CH₃)₂.

7. Dérivé de bisnaphtalimidopropyl (BNIP) de Formule I, II, III, IV, V, VI, VII ou VIII selon la revendication 1, dans lequel R₁, R₅₅, R₅₆, R₅₇, R₅₈, R₅₉, R₆₀, R₆₁, R₆₂, R₆₃ dans les molécules de la Formule VIII sont choisis indépendamment dans le groupe constitué par -H, -NH₂, -CH₃, -CH₂CH₃, -CH₂CH₂CH₃, -C(CH₃)₃, Cl, F, Br, I, -OH, -OCH₃, -OCH₂CH₃, -NHCH₃, - NHCH₂CH₃, -N(CH₃)₂, -NO₂, -COOH, -COOCH₃, -COONH₂, -COONHCH₃, -COON(CH₃)₂.

8. Dérivé de bisnaphtalimidopropyl (BNIP) de Formule I, II, III, IV, V, VI, VII ou VIII selon les revendications 5-7, dans lequel dans les molécules de la Formule VI, VII, et VIII, R₁ est - H.

9. Méthode de préparation d'un dérivé de bisnaphtalimidopropyl (BNIP) de Formule I, II, III, IV, V, VI, VII ou VIII selon l'une quelconque des revendications 1-8;
a) Réagir l'agent représenté par la Formule X avec le chlorure de paraméthyle sulfonyle en présence d'un solvant, 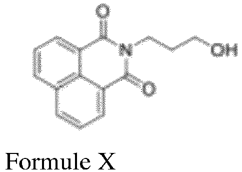
b) Mélanger le produit intermédiaire obtenu (Formule Y) avec du NaI en présence d'un solvant et ajouter un composé diaminé et une solution de K₂CO₃ dans un solvant, de préférence dans l'acétonitrile. 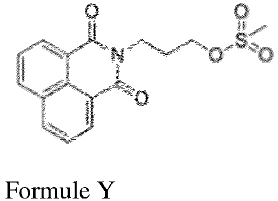
c) Isoler le produit final du mélange réactionnel.

10. Dérivé de bisnaphtalimidopropyl (BNIP) de Formule I, II, III, IV, V, VI, VII ou VIII selon l'une quelconque des revendications 1-8 pour être utilisé comme un médicament pour le traitement des maladies bactériennes, virales, parasitaires et néoplasiques.

11. Dérivé de bisnaphtalimidopropyl (BNIP) de Formule I, II, III, IV, V, VI, VII ou VIII selon la revendication 10, dans lequel la maladie parasitaire est schistosomiase, opisthorchiase, clonorchiase, dicrocoeliase, fascioliase, paragonimiase, fasciolopsiase, échinocoque, téniasis, cysticercose, diphilobotriase, sparganose, hyménolépiase, dracunculose, onchocercose, filariose, éléphantiasis, mansonellose, trichinellose, ankylostomiase, nécatoriase, ankylostomiase, ascaridose, strongyloidiase, trichuriose, entérobiase, anisakiase, helminthiase, trichostrongylus, angiostrongylus gnathostomiase, angiostrongylus, syngamiasis, hirudiniasis, acanthocephaliasis, gongylonemiasis, metastrongylosis, thelaziasis, pediculosis, pityriasis, scabies, myiasis, tungiasis, trombiculosis, scrabiasis, scrabiasis, hirudiniasis, linguatulosis, porocephaliasis, leishmaniasis, trypanosomiasis, paludisme causé par le plasmodium falciparum, paludisme causé par le plasmodium vivax, paludisme causé par le plasmodium malariae, la maladie de chagas, toxoplasmose, pneumocystose, babésiose, piroplasmose, acanthamoebose, naegleriose et/ou microsporidiose.

12. Dérivé de bisnaphtalimidopropyl (BNIP) de Formule I, II, III, IV, V, VI, VII ou VIII selon la revendication 10 dans lequel la maladie bactérienne est fièvre bubonique, fièvre pneumonique, choléra, syphilis, syphilis congénitale, glomérulonéphrite post streptococcique, fièvre puerpérale, impétigo, érysipèle, rhumatisme articulaire aigu, scarlatine, pharyngite streptococcique, septicémie, pneumonie bactérienne aiguë, méningite, otite moyenne, endométrite, septicémie néonatale, méningite néonatale, pneumonie néonatale, cystite, ostéomyélite, syndrome de choc toxique, intoxication alimentaire staphylococcique, shigellose, salmonelle, paratyphoïde, typhoïde, fièvre pourprée des montagnes Rocheuses, infections des voies urinaires, infections de la cornée, pneumonie, endocardite, méningite, gonorrhée, maladie inflammatoire pelvienne, tuberculose, lèpre, listériose, leptospirose, maladie légionnaire, ulcère peptique, infections des voies respiratoires supérieures, gastrite chronique, bronchite, arthrite septique, tularémie, diarrhée sanglante, infections des voies urinaires, endocardite, infections des voies biliaires, diphtérie, tétanos, maladie du perroquet, trachome, conjonctivite néonatale, urétrite, épididymite, prostatite, lymphogranulomatose, pneumonie atypique, syndrome de Guillain-Barre, brucellose, maladie de Lyme.

13. Dérivé de bisnaphtalimidopropyl (BNIP) de Formule I, II, III, IV, V, VI, VII ou VIII selon la revendication 10 dans lequel la maladie virale est gastro-entérite, kératoconjonctivite, pharyngite, croup, pneumonie, cystite, fièvre aphteuse, pleurodynie, méningite septique, péricardite, myocardite, lymphome de Burkitt, lymphome de Hodgkin, carcinome nasopharyngé, hépatite aiguë, hépatite chronique, cirrhose hépatique, carcinome hépatocellulaire, amygdalite, maladie des inclusions cytomégaliques, sarcome de Kaposi, maladie de Castleman, grippe, rougeole, syndrome de Reye, oreillons, verrues anogénitales, poliomyélite, rage, rubéole congénitale, rubéole, variole, zona, variole congénitale.

14. Dérivé de bisnaphtalimidopropyl (BNIP) de Formule I, II, III, IV, V, VI, VII ou VIII selon la revendication 10, dans lequel la maladie néoplasique est cancer, tel que carcinome, lymphome, blastome, sarcome, leucémie, cancer du sein, cancer de la prostate, cancer colorectal, cancer de la peau, cancer du poumon à petites cellules, cancer du poumon non à petites cellules, mésothéliome, cancer gastro-intestinal, cancer du pancréas, glioblastome, cancer de la vulve, cancer du col de l'utérus, carcinome endométrial, cancer de l'ovaire, cancer du foie, hépatome, cancer de la vessie, cancer du rein, carcinome des glandes salivaires, cancer de la thyroïde et divers cancers de la tête et du cou.

15. Composition pharmaceutique comprenant un dérivé de bisnaphtalimidopropyl (BNIP) de Formule I, II, III, IV, V, VI, VII ou VIII selon la revendication 1-8.

16. Composition pharmaceutique comprenant selon la revendication 15, comprenant au moins un autre agent actif choisi parmi les agents actifs connus qui ont des effets antiparasitaires, antibactériens, antiviraux, antinéoplasiques et/ou cytotoxiques et/ou antimétastatiques ou parmi les combinaisons comprenant deux ou trois desdites agents en plus du dérivé de bisnaphtalimidopropyl (BNIP) de Formule I, II, III, IV, V, VI, VII ou VIII.
